# EUROPEAN PATENT APPLICATION

(11) **EP 1 972 347 A1**
(43) Date of publication of application: **24.09.2008**
(21) Application number: 08250961.3
(22) Date of filing: 19.03.2008
(51) Int. Cl.: A61K 39/02, A61K 9/14, A61P 31/00, A61P 31/04

(54) **Stable vaccine powder formulations**

(30) Priority: 19.03.2007 US 918759
(71) Applicant: Becton, Dickinson and Company, Franklin Lakes, NJ 07417 (US)
(72) Inventor: Sullivan, Vincent J, Cary, NC 27519 (US); Alarcon, Jason B, Durham, NC 27703 (US); Huang, Joanne, Cary, NC 27519 (US); Ford, Brandi M, Apex, NC 27502 (US); Mikszta, John A, Durham, NC 27705 (US); Ferriter, Mathew S, Chapel Hill, NC 27516 (US); D' Souza, Ajit M, Cary, NC 27519 (US)
(74) Representative: Duckworth, Timothy John

(57) **Abstract**

The present invention is directed to methods for preparing a stable powder formulation of a vaccine. The methods comprise atomizing a liquid formulation comprising an immunogen to produce an atomized formulation, freezing the atomized formulation to produce frozen particles, and drying the frozen particles to produce dried powder particles. Pharmaceutical compositions comprising a stable powder formulation of a vaccine made in accordance with the methods of the invention are also disclosed herein. The pharmaceutical compositions are stable at high temperatures and can be reconstituted in a pharmaceutically acceptable carrier comprising an adjuvant to produce a reconstituted liquid vaccine that exhibits little or no particle agglomeration and retains immunogenicity. Methods of using the vaccine compositions for preventing and treating a disease in a subject, wherein the disease is associated with the particular immunogen, are further provided.

## Description

### FEDERALLY SPONSORED RESEARCH OR DEVELOPMENT

This invention was made with government support under contract number DAMD17-03-2-0037 awarded by the United States Medical Research and Materiel Command. The government has certain rights in the invention.

### FIELD OF THE INVENTION

The present invention relates to methods of preparing stable vaccine powder formulations, compositions prepared in accordance with these methods, and methods of using these compositions in the prevention and treatment of disease.

### BACKGROUND OF THE INVENTION

Recent world events have generated significant interest in developing stable vaccine formulations that could be use prophylactically or therapeutically to combat the use of biological compounds as bioterrorist agents. Botulinum neurotoxins (BoNTs) are among the most toxic proteins to humans and, therefore, represent a likely biological weapon for use by terrorists. Botulism is a potentially deadly neurological disorder in which BoNT binds to the synapses of motor neurons and prevents the release of the neurotransmitter acetylcholine. As a result, exposure to a BoNT can lead to blurred vision, dysphagia, general respiratory and musculoskeletal paralysis, and death caused by respiratory or cardiac failure within a few days of exposure to the toxin. Seven different serotypes of the bacterium *Clostridium botulinum* are known, and each strain produces a different form of BoNT, designated BoNT/A, B, C, D, E, F, and G. The most widely studied BoNT, BoNT/A, is synthesized in a specific C. *botulinum* strain as an approximately 150 kDa single chain protein. This single chain protein is cleaved to produce a 100 kDa heavy chain (HC) and a 50 kDa light chain (LC) linked by a disulfide bond. See, for example, Li and Singh (2000) Biochem. 39:6466-6474 and Swaminathan and Eswaramoorthy (2000) Nature Structural Biol. 7:693-699.

*Bacillus anthracis* is the causative agent of the pulmonary (i.e., inhalational), cutaneous, and gastrointestinal forms of anthrax. The possibility of creating aerosolized anthrax spores has made *B. anthracis* a bioterrorist agent of choice. Inhalational anthrax, which would result from an aerosolized or weaponized form of this bacterium, has a fatality rate of nearly 100% if not treated shortly after exposure and prior to the development of symptoms. Patients suffering from inhalational anthrax generally present initially with a high fever and chest pain that rapidly progresses to a systemic hemorrhagic pathology and cardiac or respiratory arrest. Approximately ninety strains of *B. anthracis* are known, ranging from benign strains to highly virulent strains that could be used as biological weapons. Virulent *B*. *anthracis* strains comprise a poly-D-glutamyl capsule, which is itself nontoxic but mediates the invasive stage of the disease, and a multi-component toxin. The anthrax toxin has three distinct antigenic components, each of approximately 80 kDa, designated the edema factor ("EF" or "Factor I"), the protective antigen ("PA" or "Factor II"), and the lethal factor ("LF" or "Factor III"). The protective antigen comprises the binding domain of the anthrax toxin and is so-named because it induces protective antitoxic antibodies when administered to certain mammals. Previous research has established that the lethal factor is necessary to produce the lethal effects exhibited by the anthrax toxin. The combination of only the lethal factor and the protective antigen has been shown to be lethal in experimental animals. See Bravata et al. (2006) Annals Intern. Med. 144(4):270-280; *Todor's Online Textbook of Bacteriology: Bacillus anthracis and anthrax* at textbookofbacteriology.net/Anthrax.html (University of Wisconsin-Madison Department of Microbiology); and *Brock Biology of Microorganisms* (M. Madigan and J. Martinko, eds.; Prentis Hall, 2005) for general discussions of *B. anthracis* and anthrax. Despite the significant threat of a bioterrorist attack with *B. anthracis,* only one anthrax vaccine is currently used in the U.S. and multiple doses must be administered over a period of months to elicit protective immunity.

Staphylococcal enterotoxin B (SEB) is an approximately 28 kDa enterotoxin produced by the bacterium *Staphylococcus aureus* and is traditionally associated with food poisoning resulting from unrefrigerated meats and dairy products. Classic signs of food poisoning caused by SEB are an abrupt onset of gastrointestinal symptoms that are generally self-limiting and resolve within twenty-four hours. Of greater concern in the current international climate is the fact that SEB is a potential bioterrorist agent, particularly because SEB is stable, easily aerosolized, and can cause systemic damage, multi-organ system failure, septic shock, and even death when inhaled at very high levels. Symptoms of inhalation of SEB include but are not limited to high fever, shortness of breath, severe chest pain, and, in severe cases, pulmonary edema and adult respiratory distress syndrome (ARDS). Accordingly, SEB, particularly aerosolized SEB, represents a significant bioterrorist threat.

The gram-negative bacterium *Yersinia pestis* is the causative of the plague. *Y. pestis* comprises a fraction 1 capsular antigen (i.e., "F1"), which confers antiphagocytic properties to the bacterial cells, and a V antigen that suppresses the host's innate immune response. A fusion protein comprising the two antigens, designated F1-V, has also been produced. See, for example, Santi et al. (2006) Proc. Natl. Acad. Sci. USA 103:861-866.

Three clinical forms of plague exist in humans: bubonic, septicemic, and pneumonic plague. Pneumonic plague is the most serious form of *Y. pestis* infection and occurs when the bacteria infect the lungs and cause pneumonia. Primary pneumonic plague results from direct inhalation of the *Y. pestis* bacteria, such as by airborne transmission from an infected person to an uninfected individual or by intentional release of aerosolized bacteria (e.g., a bioterrorist attack). Kool (2005) Healthcare Epidemiology 40:1166-1172. Pneumonic plague has an incubation period of approximately 1-6 days and is characterized by, for example, the sudden onset of severe headache, chills, malaise, and increased respiratory and heart rates. *Id.* These symptoms rapidly progress to pneumonia and may ultimately lead to respiratory failure and death if left untreated. See Josko (2004) Clin. Lab. Sci. 17:25-29. Appropriate antibiotics, if administered in a timely fashion (i.e., within approximately 20 hours of the onset of the disease) reduce mortality rates, but fatalities resulting from pneumonic plague remain high and could be particularly great in the event of a bioterrorist event in which numerous individuals could be exposed and the national stockpile of antibiotics could be rapidly depleted.

The CDC and Homeland Security have deemed *Y. pestis* a logical candidate for a potential bioterrorist weapon, one which poses a particularly dangerous threat because of: 1) its natural occurrence on every continent, 2) the ease of its dissemination from wild and domesticated animal reservoirs, as well as man-made devices, 3) a lack of current experience with its clinical presentation coupled possibly with physician complacency in this era of readily available antibiotics, 4) the ability to mass produce the bacteria, 5) the ease by which genetically modified, antibioticresistant strains can be produced and aerosolized, and 6) the fact that primary pneumonic plague can be spread from person to person via inhalation of contaminated aerosol droplets. See, for example, Finegold et al. (1968) Am. J. Path. 53:99-114; Walker (1968) Curr. Top. Micro. Immun. 41:23-42; Walker (1968) J. Infect. Dis. 118:188-96; Beebe and Pirsch (1958) Appl. Microbiol. 6:127-138; Williams et al. (1994) J. Wildlife Dis. 30:581-585; Watson et al. (2001) Veter. Path. 38:165-172; and Green et al. (1999) Med Micro. 23:107-113.

The potential use of a BoNT, a virulent strain of *B. anthracis,* a Staphylococcal enterotoxin (e.g., SEB), or *Y. pestis* as bioterrorist agents makes the development of stable powder vaccines against a BoNT, anthrax, SEB, and/or *Y. pestis* advantageous, particularly if such vaccines could be administered by a variety of techniques, including minimally-invasive methods, and by medical or non-medical personnel. Stabilized BoNT, anthrax, SEB, and *Y. pestis* vaccine powder formulations could be readily reconstituted to permit the prophylactic immunization of first responders, military personnel, and possibly even the general population in the event or threat of a bioterrorist attack. Stable polyvalent vaccines that provide protective immunity against a plurality of bioterrorist agents would be particularly advantageous.

Vaccines typically contain at least one adjuvant to enhance a subject's immune response to the immunogen. Aluminum salts are frequently used as adjuvants to boost the immunogenicity of vaccines. The application of traditional approaches for stabilizing liquid biological products as stable, dried powder formulations (e.g., lyophilization, spray-freeze drying, etc.), however, has typically been problematic when the immunogen is "pre-adsorbed" to the aluminum adjuvant. In particular, alum-adsorbed vaccines generally exhibit agglomeration, decreased immunogen concentration, and loss of immunogenicity when subjected to conventional lyophilization, freezing, and freeze-drying processes. See, for example, Maa et al. (2003) J. Pharm. Sci. 92:319-332; Diminsky et al. (1999) Vaccine 18:3-17; Alving et al. (1993) Ann. NY Acad. Sci. 690:265-275; and Warren et al. (1986) Ann. Rev. Immunol. 4:369-388, all of which are herein incorporated by reference. The use of conventional methods to produce stable powdered formulations of alum-adsorbed vaccines that can be reconstituted without a loss of stability and immunogenicity has been largely unsuccessful. Therefore, methods are needed in the art for the production of readily reconstitutable stable vaccine powder formulations, wherein the aluminum adjuvant can be added at the time of reconstitution to produce efficacious liquid vaccines that exhibit little or no particle agglomeration, loss of immunogenicity, or immunogen concentration. Such methods would facilitate long-term vaccine storage, extend the shelf-life of these vaccines, permit their use in areas where refrigerated storage and transportation are unavailable, allow for vaccine administration to subjects by a variety of techniques (e.g., potentially minimally invasive administration methods that would not require medical personnel) and, particularly with respect to bioterrorist agents, facilitate stockpiling of the vaccine.

### BRIEF SUMMARY OF THE INVENTION

The present invention is directed to methods for preparing a stable vaccine powder formulation. The methods comprise atomizing a liquid formulation comprising at least one immunogen to produce an atomized formulation, freezing the atomized formulation to produce frozen particles, and drying the frozen particles to produce dried powder particles. Drying of the frozen particles may be performed at about atmospheric pressure, particularly in the presence of vibration, internals, and/or mechanical stirring. The methods of the invention permit the production of vaccines in powder form that are stable even when subjected to non-optimal conditions (e.g., high temperatures, freeze-thaw, etc.). The vaccine powder formulations disclosed herein are generally reconstituted in a diluent comprising an adjuvant, such as, for example an aluminum adjuvant or lipopolysaccharide (LPS), to produce reconstituted liquid vaccines that may exhibit little or no particle agglomeration, display no significant decrease in immunogen concentration, retain a substantial level of immunogenicity and/or antigenicity, and maintain protective efficacy against the disease or disorder of interest. Moreover, the powder and reconstituted vaccine formulations of the invention may be suitable for administration by medical or non-medical personnel by a variety of methods, particularly minimally invasive administration techniques. Pharmaceutical compositions comprising stable vaccine powder formulations (or reconstituted liquid forms thereof) prepared by the above methods and methods of using these compositions for preventing and treating particular diseases, disorders, and the symptoms thereof are also provided.

### BRIEF DESCRIPTION OF THE DRAWINGS

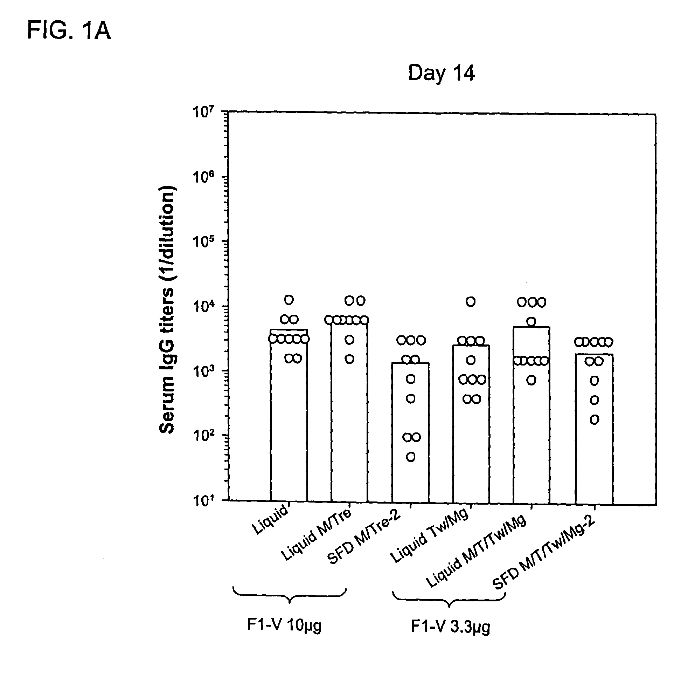

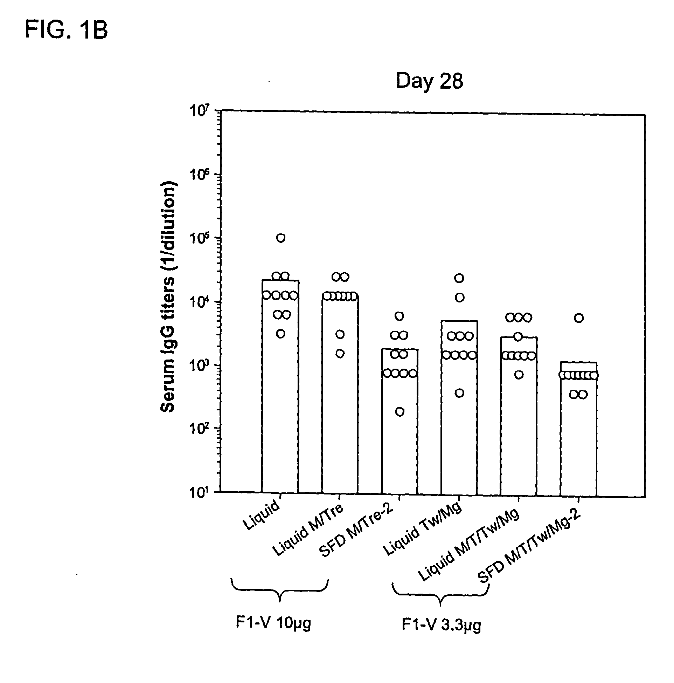

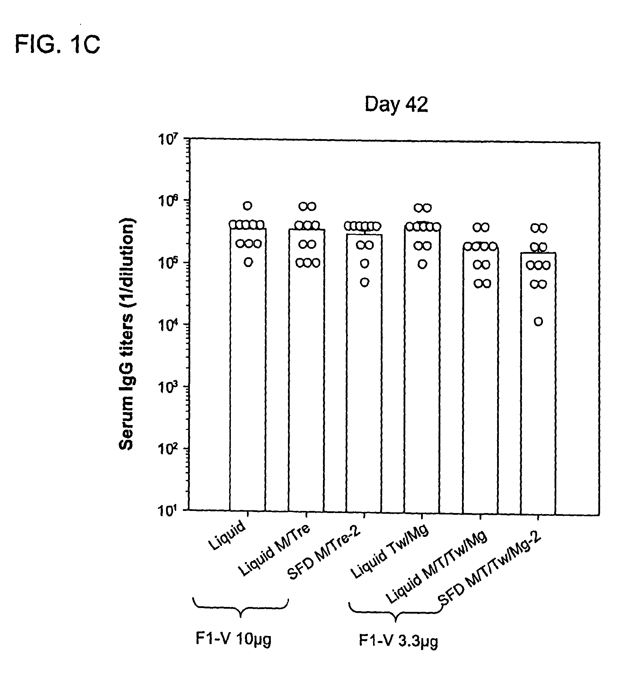

Figure 1 provides the F1-V specific IgG antibody titers (1/dilution) in serum from mice immunized with the specified liquid or reconstituted SFD vaccine formulations at day 14 (Figure 1A), day 28 (Figure 1B), and day 42 (Figure 1C). Mice were immunized at day 0 and day 28. Circles in these figures represent serum antibody titers for individual mice, and the bars provide the mean antibody titer for all of the animals in a particular test group. Additional experimental details are provided in Example 1.

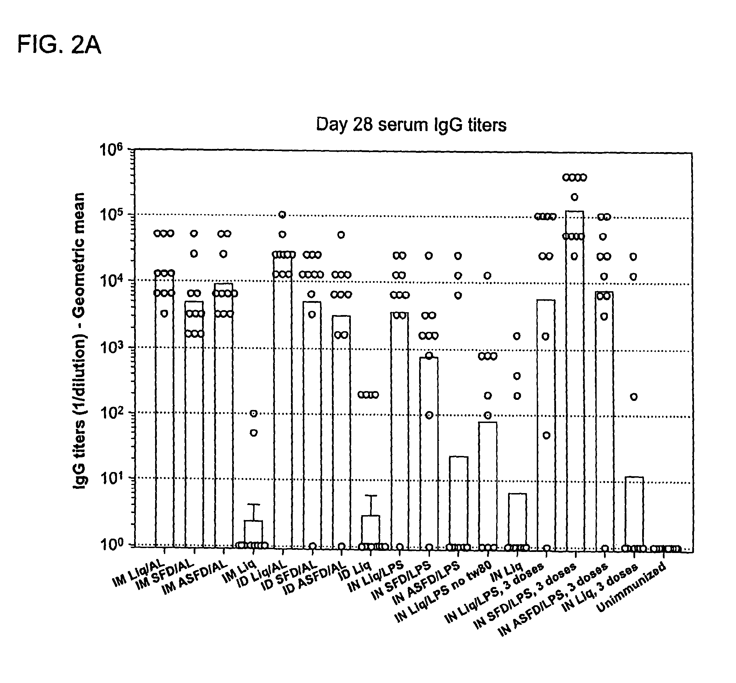

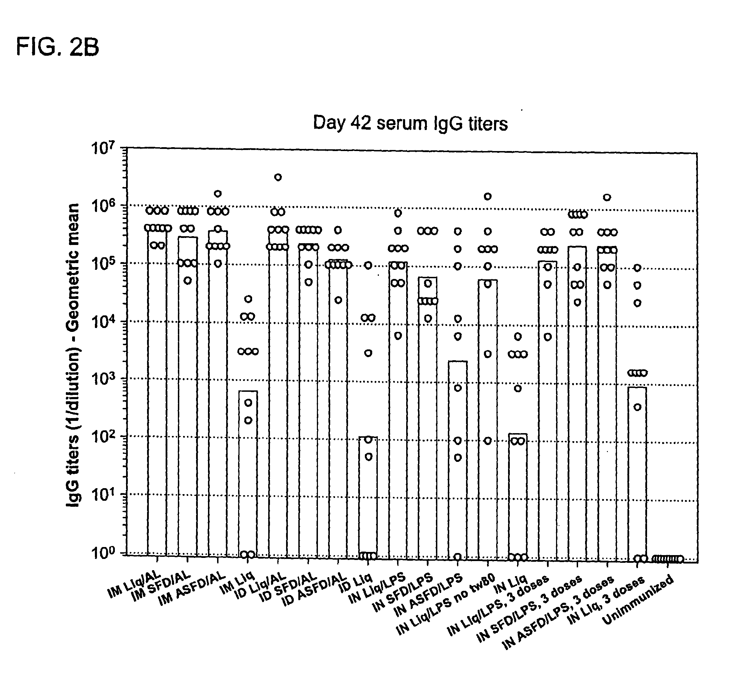

Figure 2 provides the F1-V specific IgG antibody titers (1/dilution) in serum from mice immunized with the specified liquid, reconstituted SFD, or reconstituted ASFD vaccine formulations at day 28 (Figure 2A) and day 42 (Figure B). Mice were immunized at day 0 and day 28, with the exception of four test groups receiving a third immunization intranasally on day 14. Circles in these figures represent serum antibody titers for individual mice, and the bars provide the mean antibody titer for all of the animals in a particular test group. Additional experimental details are provided in Example 2.

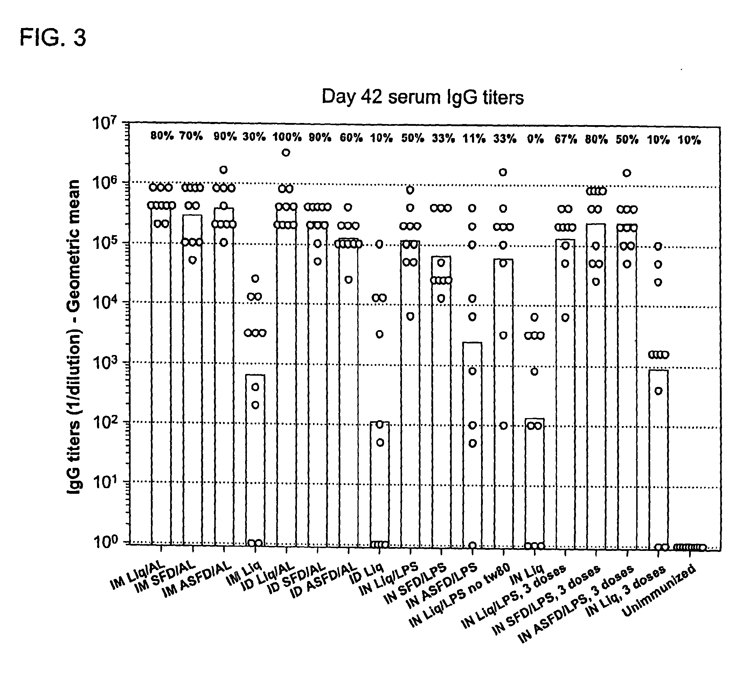

Figure 3 provides the F1 specific IgG antibody titers (1/dilution) in serum from mice immunized with the specified liquid, reconstituted SFD, or reconstituted ASFD vaccine formulations at day 42. Mice were immunized at day 0 and day 28, with the exception of four test groups receiving a third immunization intranasally on day 14. Circles in these figures represent serum antibody titers for individual mice, and the bars provide the mean antibody titer for all of the animals in a particular test group. The percentages indicate survival rates for each test group following lethal challenge with *Y. pestis* on day 56. Additional experimental details are provided in Example 2.

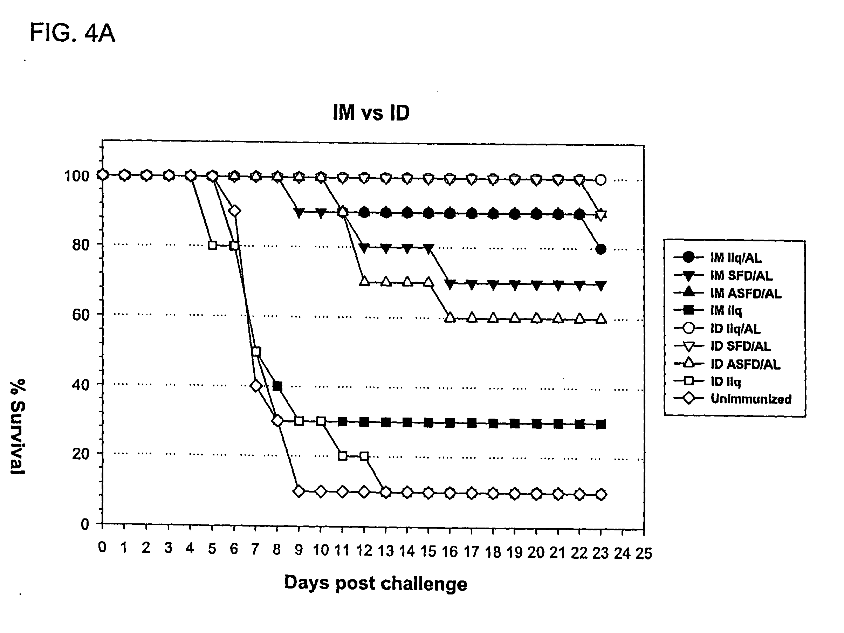

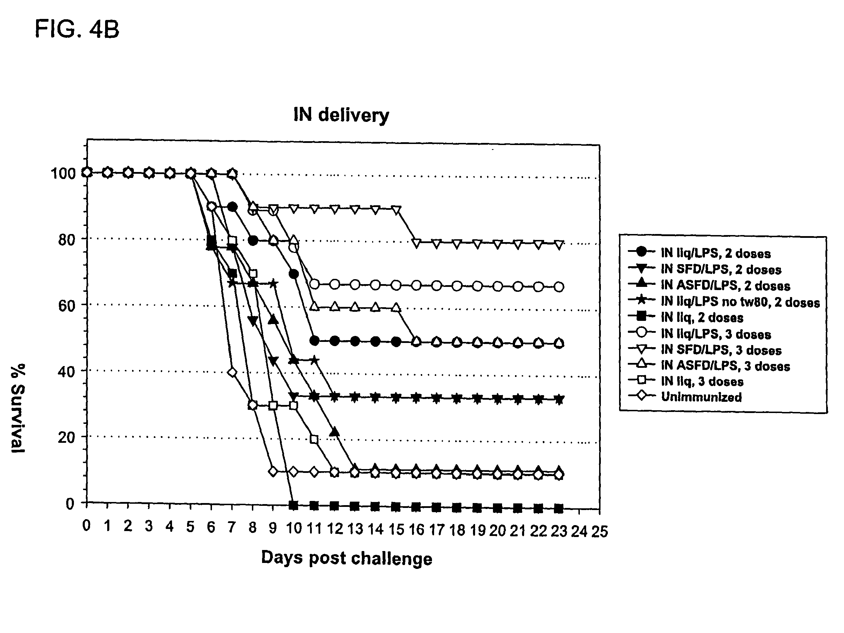

Figure 4 provides the survival rates for mice immunized with the specified vaccine formulations and then challenged subcutaneously at day 56 with a lethal dose (1000 x LD₅₀) of *Y. pestis.* Mice were monitored daily for morbidity and mortality post-challenge. A comparison of the vaccine formulations injected intradermally (ID) and intramuscularly (IM) is set forth in Figure 4A. The survival rates following intranasal delivery (IN) of the specified vaccines is provided in Figure 4B. Experimental details are provided in Example 3.

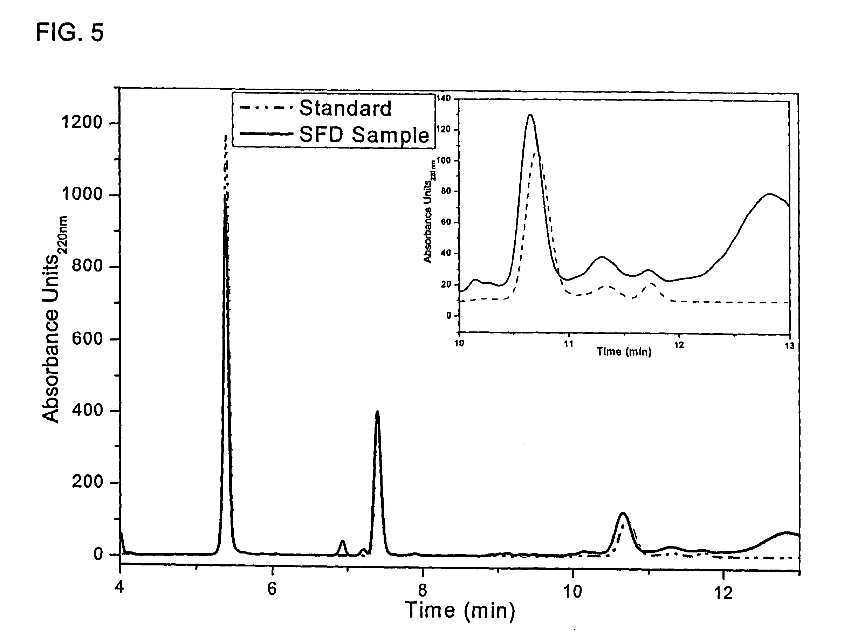

Figure 5 provides a chromatogram from reverse-phase HPLC analysis of a reconstituted SFD polyvalent vaccine comprising rPA, rSEB, rF1-V, and rBoNT/A (solid line) and a standard, unprocessed sample containing these antigens (dashed line). All four of the antigens were soluble upon reconstitution and were completely recovered following SFD. Additional experimental details are provided in Example 3.

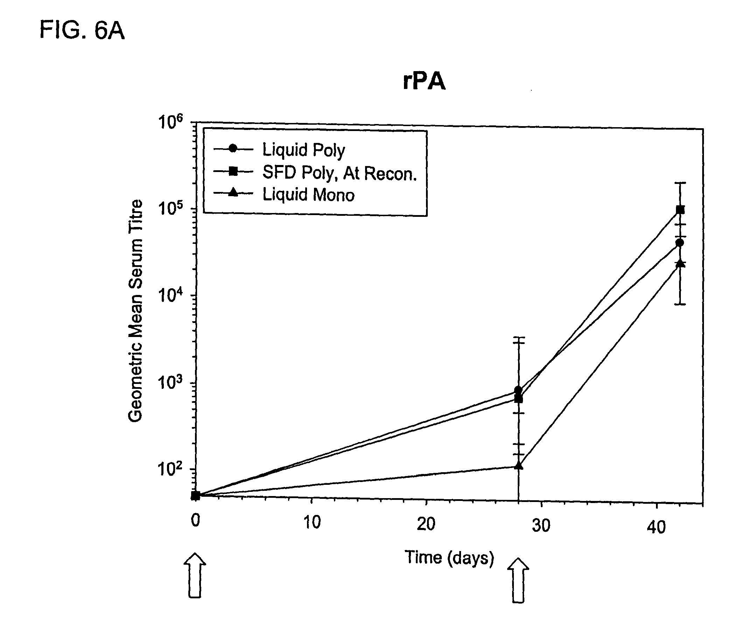

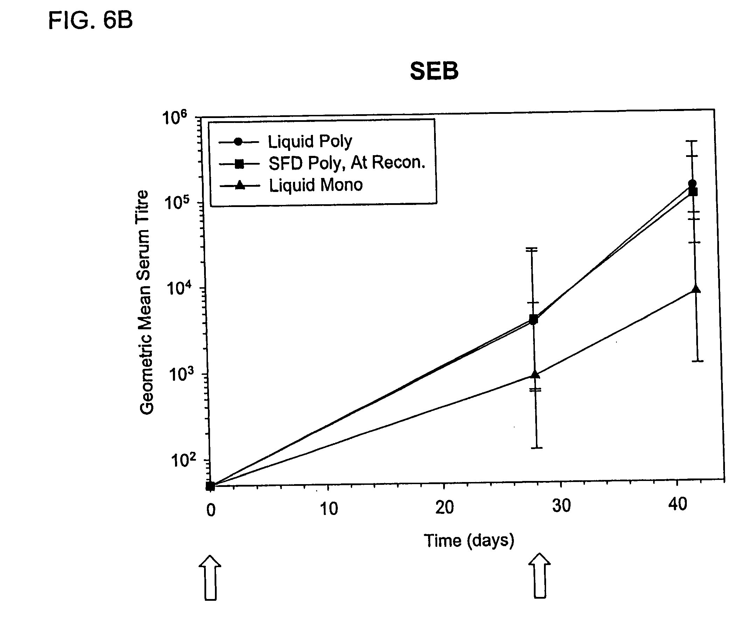

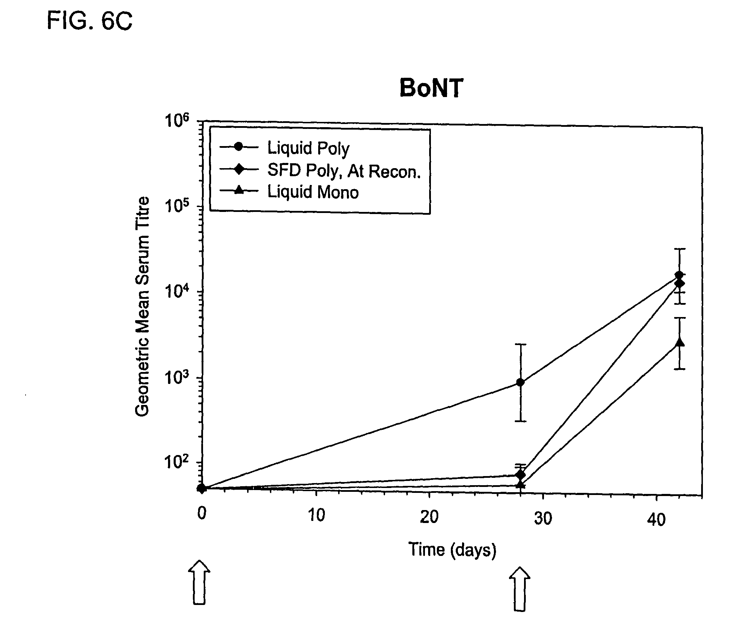

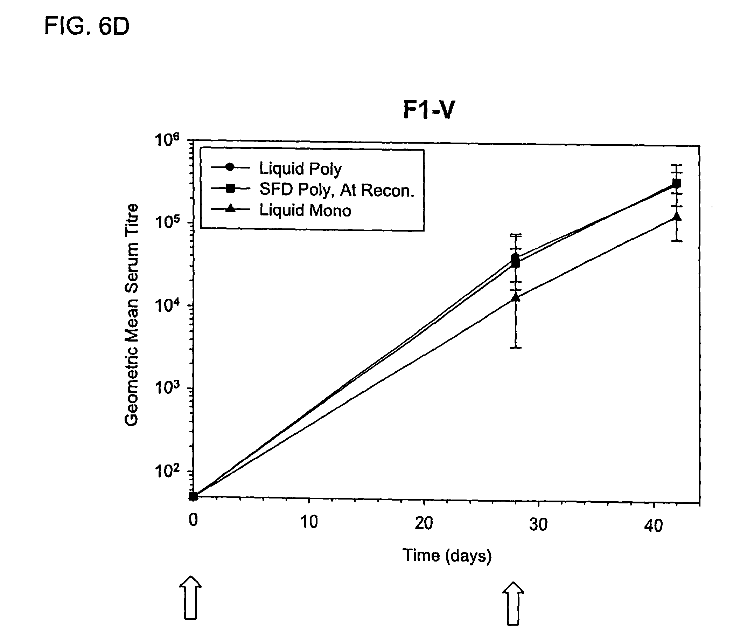

Figure 6 provides the geometric mean serum antibody titers from mice immunized with either polyvalent liquid vaccine pre-adsorbed to aluminum hydroxide adjuvant ("Liquid Poly"), reconstituted SFD powder vaccine to which aluminum hydroxide was added at time of reconstitution ("SFD Poly, At Recon."), or monovalent liquid vaccine control ("Liquid Mono"). Serum samples were screened for antibodies against constituent antigens of the polyvalent vaccine, i.e. recombinant Protective Antigen (rPA) from *Bacillus anthracis* (Figure 6A), Staphyloccocal Enterotoxin B (SEB) from *Staphlycoccus aureus* (Figure 6B), Botulinum Neurotoxin (BoNT) from *Clostridium botulinum* (Figure 6C), and F1-V fusion protein from *Yersinia pestis* (Figure 6D). Mice were immunized at days 0 and 28, as indicated by arrows, and blood was obtained for antibody analysis at days 0, 28 and 42. Additional experimental details are provided in Example 3.

### DETAILED DESCRIPTION OF THE INVENTION

The present invention is directed to methods for preparing a stable vaccine powder formulation of a vaccine of interest, such as a Botulinum neurotoxin (BoNT), anthrax (i.e., *B. anthracis),* plague (i.e., *Y. pestis),* or Staphylococcal enterotoxin (e.g., Staphylococcal enterotoxin B (SEB) from *S*. *aureus)* vaccine. Stable polyvalent vaccine powder formulations that comprise a plurality of immunogens and provide protective immunity against multiple disease-causing agents or toxins are further provided. The stable vaccine powder formulations disclosed herein are generally prepared by spray-freeze-drying (SFD) or atmospheric spray-freeze-drying (ASFD) techniques, such as those described in U.S. Patent Application Publication No. 2003/0180755 and Jiang et al. (2006) J. Pharm. Sci. 95:80-96, both of which are incorporated by reference in their entirety. The vaccine powder compositions made in accordance with the present methods may be reconstituted to produce an efficacious liquid vaccine. Methods of using the compositions of the invention are also encompassed by the present disclosure.

The disclosed vaccine powder formulations are stable when stored at high temperatures (i.e., above conventional refrigeration temperatures) and/or subjected to freeze-thaw. The vaccine powder formulations can be readily reconstituted in a diluent to produce a reconstituted liquid vaccine that exhibits little or no particle agglomeration, displays no significant decrease in immunogen concentration, retains a substantial level of immunogenicity or antigenicity, and/or exhibits a significant level of protection against the disease-causing pathogen or toxin of interest (i.e., "protective efficacy" or "protective immunity"). Methods for preparing reconstituted liquid vaccines are also disclosed and generally involve the addition of a liquid carrier comprising an adjuvant (e.g., an aluminum adjuvant or lipopolysaccharide (LPS)) to the dried vaccine powder at the time of reconstitution. Methods for using the vaccine powder compositions (or reconstituted liquid formulations thereof) in the prevention or treatment of particular diseases, disorders, or symptoms associated with exposure to a particular disease-causing pathogen or toxin are further provided. Moreover, the readily reconstitutable nature of the stable vaccine powder formulations disclosed herein may permit administration of the reconstituted vaccines by a variety of methods. In certain aspects of the invention, a reconstituted vaccine may be administered by minimally invasive techniques, with or without the assistance of medically trained personnel. For example, a reconstituted liquid vaccine of the invention, particularly an anthrax vaccine, a BoNT vaccine, more particularly a BoNT/A vaccine, a plague vaccine, more particularly an F1-V plague vaccine, or a Staphylococcal enterotoxin vaccine, more particularly an SEB vaccine, may be administered intradermally by a microneedle.

Methods for preparing a stable powder formulation of a vaccine of interest comprise providing a liquid formulation comprising at least one immunogen, wherein the liquid formulation does not contain an aluminum adjuvant, atomizing the liquid formulation to produce an atomized formulation, freezing the atomized formulation to produce frozen particles, and drying the frozen particles to produce dried powder particles. Such methods may be referred to herein as "spray-freeze-drying (SFD)." See, for example, Jiang et al. (2006) J. Pharm. Sci. 95:80-96; Maa et al. (2003) J. Pharm. Sci. 92:319-332; and U.S. Patent Application Publication No. 2003/0180755, all of which are herein incorporated by reference. The immunogens present in the liquid formulations used in the SFD and ASFD methods disclosed herein may be referred to at times as "non-alum-adsorbed" to expressly indicate that the liquid formulation to be processed does not contain an aluminum adjuvant prior to SFD or ASFD. In a particular aspect of the invention, the claimed methods for preparing a stable powder formulation of a vaccine of interest comprise providing a liquid formulation comprising at least one non-alum-adsorbed immunogen, atomizing the liquid formulation to produce an atomized formulation, freezing the atomized formulation to produce frozen particles, and drying the frozen particles at about atmospheric pressure to produce dried powder particles. The drying step may be performed in the presence of vibration, internals, mechanical stirring, or a combination thereof. This method of producing powder formulations may be referred to as "atmospheric spray-freeze-drying (ASFD)." See U.S. Patent Application Publication No. 2003/0180755.

"Alum-adsorbed vaccine" refers to an immunogenic composition that comprises an immunogen and an aluminum adjuvant, particularly wherein the immunogen is adsorbed onto the aluminum adjuvant. Conventional liquid formulations of alum-adsorbed vaccines have been shown to lose immunogenicity and to aggregate when subjected to traditional lyophilization, freezing, and freeze-drying techniques that are used to facilitate long-term storage. See, for example, Maa et al. (2003) J. Pharm. Sci. 92:319-332; Diminsky et al. (1999) Vaccine 18:3-17; Alving et al. (1993) Ann. NY Acad. Sci. 690:265-275; and Warren et al. (1986) Ann. Rev. Immunol. 4:369-388, all of which are herein incorporated by reference. As a result, alum-adsorbed vaccines must generally be stored and transported as liquid formulations under refrigerated conditions (e.g., at about 2°C to about 8°C). The methods of the present invention, however, permit the production of a stable powder formulation of a vaccine that does not contain an aluminum adjuvant, such as BoNT, anthrax, plague (e.g., F1-V), or Staphylococcal enterotoxin (e.g., SEB) vaccine. Such vaccines can be stored under non-optimal conditions (e.g., non-refrigerated conditions) and can be reconstituted in a suitable liquid carrier, particularly a liquid carrier that comprises an adjuvant (e.g., an aluminum adjuvant or LPS) to produce a reconstituted liquid vaccine that exhibits little or no particle agglomeration, retains immunogenicity/immunogenicity, and maintains protective efficacy against the disease, toxin, or symptoms associated therewith. "Non-optimal conditions" or "non-optimal storage conditions" as used herein generally refer to conditions such as storing the vaccine composition at high temperatures, subjecting the vaccine formulation to one or more freeze-thaw cycles, and storing the vaccine composition for prolonged time periods. By "high temperature" is intended temperatures above the refrigeration conditions traditionally recommended for storage of liquid vaccine formulations and may include, for example, temperatures of 10°, 20°, 30°, 40°, 50°, 55°C or higher.

By "powder" or "powder formulation" is intended a composition that consists of substantially solid, free-flowing particles. A "stable powder formulation" produced by an SFD or ASFD method of the invention maintains substantial structural integrity (e.g., displays little or no agglomeration, maintains a substantial amount of the original immunogen concentration, etc.) and retains a substantial level of immunogenicity, antigenicity, and/or protective efficacy relative to that of the original liquid formulation. In particular aspects of the invention, the vaccine powder formulation is stable even when subjected to storage under non-optimal conditions (e.g., high temperatures, freeze-thaw cycles, long-term storage, etc.). For example, a stable vaccine powder formulation of the invention may be stored under non-optimal conditions and reconstituted to produce a liquid vaccine formulation, wherein the dried vaccine powder is reconstituted in a suitable diluent comprising an adjuvant (e.g., an aluminum adjuvant or LPS) and the reconstituted liquid vaccine exhibits little or no particle agglomeration, maintains a substantial amount of the original immunogen concentration, and further retains a substantial level of immunogenicity and/or antigenicity, as described further herein below. Stability of a vaccine composition may be assessed by measuring, for example, the rate of sedimentation, which corresponds to the extent of particle agglomeration, and the concentration of immunogen present in the reconstituted liquid vaccine. In particular, the rate of sedimentation and concentration of immunogen of the reconstituted liquid vaccine may be compared with that of the original liquid formulation (i.e., the liquid formulation comprising the non-alum-adsorbed immunogen prior to atomization). Standard assays for measuring the rate of sedimentation, concentration of immunogen, immunogenicity, and antigenicity are known in the art. Protective efficacy may be assessed by, for example, evaluating the survival rates of immunized and non-immunized subjects following challenge with a disease-causing pathogen or toxin associated with a particular immunogen of interest. With regard to the anthrax vaccines of the invention, protective immunity may be analyzed, for example, via anthrax lethal toxin neutralization assays.

The liquid formulations that are atomized in accordance with the SFD oR ASFD methods of the invention include at least one non-alum-adsorbed immunogen (i.e., the liquid formulation to be processed by SFD or ASFD does not contain an aluminum adjuvant). An "immunogen" is any naturally occurring or synthetic substance that induces an immune response in a subject. A liquid formulation comprising more than one immunogen may be used in the practice of the invention and is referred to generally as a "polyvalent" or "multivalent" vaccine. As used herein, the term "immunogenicity" refers to the ability of a substance to induce an immune response when administered to a subject (e.g., a cellular immunogen-specific immune response or a humoral antibody response). The immunogens of the invention may be associated with or derived from any pathogen of interest and include, for example, whole cells, viral particles (e.g., partially or completely inactivated viruses), polypeptides, polynucleotides, carbohydrates, lipids, lipoproteins, glycoproteins, and polysaccharides. In particular aspects of the invention, the immunogen comprises a botulism immunogen including, for example, a Botulinum neurotoxin such as BoNT/A, B, C, D, E, F, or G. The BoNT immunogen of the invention is typically a BoNT/A antigen, more particularly the BoNT/A heavy chain (HC). The immunogens of the invention also include *B. anthracis* antigens, particularly *B. anthracis* toxins or components thereof, more particularly the *B. anthracis* protective antigen (PA). In certain embodiments, the immunogen is a recombinant *B. anthracis* Protective Antigen (rPA). *Y. pestis* antigens of the invention include, for example, the F 1 antigen, the V antigen, and, particularly, the F1-V fusion protein antigen. Immunogens further include antigens of Staphylococcal enterotoxins, such as SEB, particularly recombinant SEB (rSEB). In some aspects of the invention, multiple immunogens may be used to produce a polyvalent or multivalent vaccine. Such polyvalent vaccines may comprise, for example, an anthrax antigen (e.g., rPA), a Staphylococcal enterotoxin antigen (e.g., rSEB), a BoNT antigen (e.g., BoNT/A), and a *Y. pestis* antigen (e.g., rF1-V). Recombinantly produced immunogens and variants or fragments of an immunogen of interest, as defined herein below, may be used to practice the present invention.

Any suitable immunogen as defined herein may be employed. The immunogen may be a viral immunogen. The immunogen may therefore be derived from members of the families *Picomaviridae* (e.g. polioviruses, etc.); *Caliciviridae; Togaviridae* (e.g., rubella virus, dengue virus, etc.); *Flaviviridae; Coronaviridae; Reoviridae; Birnaviridae; Rhabodoviridae* (e.g. rabies virus, etc.); *Filoviridae; Paramyxoviridae* (e.g. mumps virus, measles virus, respiratory syncytial virus, etc.); *Orthomyxoviridae* (e.g. influenza virus types A, B and C, etc.); *Bunyaviridae; Arenaviridae; Retroviradae* (e.g. HTLV-I; HTLV-II; HIV-1 and HIV-2); and simian immunodeficiency virus (SIV) among others.

Alternatively, viral immunogens may be derived from papillomavirus (e.g. HPV); a herpesvirus; a hepatitis virus, e.g. hepatitis A virus (HAV), hepatitis B virus (HBV), hepatitis C (HCV), the delta hepatitis virus (HDV), hepatitis E virus (HEV) or hepatitis G virus (HGV); and the tick-borne encephalitis viruses. See, e.g., Virology 3rd Edition (W. K. Joklik ed. 1988) and Fundamental Virology, 2nd Edition (B. N. Fields and D. M. Knipe, eds. 1991) for a description of these viruses.

Bacterial immunogens for use in the invention can be derived from organisms that cause anthrax, botulism, plague, diphtheria, cholera, tuberculosis, tetanus, pertussis, meningitis and other pathogenic states, including, e.g., *Meningococcus* A, B and C, *Haemophilus* influenza type B (HIB), *Helicobacter pylori, Vibrio cholerae, Escherichia coli, Campylobacter, Shigella, Salmonella, Streptococcus sp., Staphylococcus sp, Clostridium botulinum, Bacillus anthracis,* and *Yersinia pestis.* A combination of bacterial immunogens may be provided in a single composition comprising, for example, diphtheria, pertussis and tetanus immunogens. Suitable pertussis immunogens are pertussis toxin and/or filamentous haemagglutinin and/or pertactin, alternatively termed P69. An anti-parasitic immunogen may be derived from organisms causing malaria and Lyme disease. In certain aspects of the invention, the bacterial immunogen is selected from the group consisting of recombinant *Staphylococcus* enterotoxin B (rSEB), *Bacillus anthracis* recombinant Protective Antigen (rPA), recombinant *Clostridium botulinum* neurotoxin, and *Yersinia pestis* F1-V fusion protein. In particular embodiments, combinations of the above immunogens are utilized in the practice of the invention to produce multivalent vaccines.

Immunogens for use in the present invention can be produced using a variety of methods known to those of skill in the art. In particular, the immunogens can be isolated directly from native sources, using standard purification techniques. Alternatively, whole killed, attenuated or inactivated bacteria, viruses, parasites or other microbes may be employed. Yet further, immunogens can be produced recombinantly using known techniques.

Immunogens for use herein may also be synthesized, based on described amino acid sequences, via chemical polymer syntheses such as solid phase peptide synthesis. Such methods are known to those of skill in the art. See, e.g. J. M. Stewart and J. D. Young, Solid Phase Peptide Synthesis, 2nd Ed. (Pierce Chemical Co., Rockford, III., (1984)) and G. Barany and R. B. Merrifield, The Peptides: Analysis, Synthesis, Biology, Vol. 2 (E. Gross and J. Meienhofer, eds., Academic Press, New York (1980)), for solid phase peptide synthesis techniques; and M. Bodansky, Principles of Peptide Synthesis, (Springer-Verlag Berlin (1984)) and The Peptides: Analysis, Synthesis, Biology, Vol. 1 E. Gross and J. Meienhofer, eds.) for classical solution synthesis.

In certain aspects of the invention, the liquid formulation comprising a non-alum-adsorbed immunogen may be a commercially available liquid vaccine that is to be formulated as a stable dry powder. A liquid formulation of any non-alum-adsorbed vaccine (i.e., one that does not contain an aluminum adjuvant) may be used to practice the invention. Such vaccines include but are not limited to *Haemophilus* b conjugate vaccines (e.g., Pedvax (Merck); ActHIB (Sanofi-Pasteur)), measles vaccines (e.g., Attenuvax (Merck)), influenza vaccines (e.g., Fluvirion (Novartis); FluMist (MedImmune); Fluzone (Sanofi-Pasteur); Fluarix (GlaxoSmithKline)), rabies vaccines (e.g., Imovax (Sanofi-Pasteur)), polio vaccines (e.g., IPOL (Sanofi-Pasteur)), JE vaccines (e.g., JE-VAX (Sanofi-Pasteur)), meningococcal polysaccharide diptheria toxoid vaccines (e.g., Menactra (Sanofi-pasteur)), meningcoccal polysaccharide vaccines (e.g., Menommune (Sanofi-Pasteur)), measles/mumps/rubella vaccines (e.g., MMR-II (Merck)), mumps vaccines (e.g., Mumpsvax (Merck)), pneumonia vaccines (e.g., Pneumovax-23 (Merck)), typhoid vaccines (e.g., Typhim Vi (Sanofi-Pasteur)), and varicella vaccines (e.g., Varivax (Merck)).

Use of the term a "polynucleotide" or "polynucleotide sequence" is not intended to limit the present invention to polynucleotides comprising DNA. One of skill in the art will appreciate that polynucleotide molecules can comprise ribonucleotides, deoxyribonucleotides, and combinations thereof. A "DNA" refers to the polymeric form of deoxyribonucleotides (adenine, guanine, thymine, or cytosine) in its either single stranded form, or a double-stranded helix. This term refers only to the primary and secondary structure of the molecule, and does not limit it to any particular tertiary forms. Thus, this term includes double-stranded DNA found, *inter alia,* in linear DNA molecules (e.g., restriction fragments), viruses, plasmids, and chromosomes. In discussing the structure of particular double-stranded DNA molecules, sequences may be described herein according to the normal convention of giving only the sequence in the 5' to 3' direction along the nontranscribed strand of DNA (i.e., the strand having a sequence homologous to the mRNA). The terms "polynucleotide" and "nucleic acid" may be used interchangeably herein.

The terms "polypeptide," "peptide," and "protein" are used interchangeably herein to refer to a polymer of amino acid residues. The terms apply to amino acid polymers in which one or more amino acid residues is an artificial chemical analogue of a corresponding naturally occurring amino acid, as well as to naturally occurring amino acid polymers.

The polypeptides and polynucleotides used in the practice of the invention can be naturally occurring or recombinantly produced in accordance with routine molecular biology techniques. Variants and fragments of immunogens comprising polypeptides (e.g., BoNT/A, *B. anthracis* rPA, rSEB, or rF1-V) or polynucleotides are also encompassed by the present invention. "Variants" refer to substantially similar sequences. A variant of an amino acid or nucleotide sequence of the invention will typically have at least about 65%, 70%, 75%, 80%, 85%, 90%, 91%, 92%, 93%, 94%, 95%, 96%, 97%, 98%, 99% or more sequence identity with the reference sequence. In particular embodiments, a variant of an immunogenic polypeptide of the invention will retain the biological activity of the full-length polypeptide and hence be immunogenic. Methods for generating variant sequences are well known in the art as are methods for determining percent identity of polypeptide or polynucleotide sequences, e.g. BLAST.

The term "fragment" refers to a portion of a polypeptide or polynucleotide comprising a specified number of contiguous amino acid or nucleotide residues. In particular embodiments, a fragment of an immunogenic polypeptide of the invention may retain the biological activity of the full-length polypeptide and hence be immunogenic. Fragments of a polynucleotide may encode protein fragments that retain the biological activity of the protein and hence be immunogenic. Fragments of the polypeptides and polynucleotides of the invention can be of any length provided they have the desired attributes (e.g., immunogenicity). Methods for generating fragments of a polypeptide or a polynucleotide are known in the art.

The liquid formulations of the invention that are processed by the SFD or ASFD methods disclosed herein comprise an immunogen that is not adsorbed onto an aluminum adjuvant (i.e., a non-alum-adsorbed immunogen). Although the liquid formulations to be processed by the SFD or ASFD methods described herein do not contain an aluminum adjuvant, they may optionally include one or more non-alum adjuvant agents (e.g., LPS). The term "adjuvant" refers to a compound or mixture that enhances the immune response to an immunogen. An adjuvant can serve as a tissue depot that slowly releases the immunogen and also as a lymphoid system activator that non-specifically enhances the immune response (Hood et al., Immunology, Second Ed., 1984, Benjamin/Cummings: Menlo Park, California). Generally, the adjuvants used in the practice of the invention are pharmaceutically acceptable. Such pharmaceutically acceptable adjuvants are well known in the art.

Exemplary non-aluminum adjuvants include, but are not limited to, complete Freund's adjuvant, incomplete Freund's adjuvant, saponin (and derivatives thereof), mineral gels, surface active substances such as lysolecithin, pluronic polyols, polyanions, peptides, oil or hydrocarbon emulsions, keyhole limpet hemocyanins, dinitrophenol, and potentially useful human adjuvants such as BCG *(bacille Calmette-Guerin)* and *Corynebacterium parvum.* Other adjuvants of interest include CpG DNA, GM-CSF, IL-4, IL-7, IL-12, monophosphoryl lipid A (MPL), 3-Q-desacyl-4'-monophosphoryl lipid A (3D-MLA), IL-1beta 163-171 peptide (Sclavo Peptide), 25-dihydroxyvitamin D3, calcitonin-gene regulated peptides, dehydroepiandrosterone (DHEA), N-Acetylglucosaminyl- (P1-4)-N-acetylmuramyl-L-alanyl-D-glutamine (GMDP), dimethyl dioctadecyla or disteary ammonium bromide (DDA), Zinc L-proline, formylated-Met-Leu-Phe (fMLP), N-acetyl muramyl-L-threonyl-D-isoglutamine (Threonyl-MDP), N-acetyl-L-alanyl-D-isoglutaminyl-L-alanine-2-(1,2-dipalmitoyl-sn-glycero-3-(hydroxy-phosphoryloxy) ethylamide monosodium salt (MTP-PE), Nac-Mur-L-Ala-D-Gln-OCH3, Nac-Mur-L-Thr-D-isoGln-sn-glycerol dipalmitoyl, Nac-Mur-D-Ala-D-isoGln-sn-glycerol dipalmitoyl, 1-(2-methypropyl)-1*H*-imidazo[4,5-*c*]quinolin-4-amine, 4-Amino-otec-dimethyl-2-ethoxymethyl-1*H-*imidazo[4,5-c]quinoline-1-ethanol, N-acetylglucosaminyl-N-acetylmuramyl-L-Ala-D-isoGlu-L-Ala-glycerol dipalmitate (DTP-GDP), N-acetylglucosaminyl-N-acetylinuramyl-L-Ala-D-isoGlu-L-Ala-dipalmitoxy propylamide (DTP-DPP), 7-allyl-8-oxoguanosine, poly-adenylic acid-poly-uridylic acid complex, MIP-1a, MIP-3 a, dibutyl phthalate, dibutyl phthalate analogues and C5a.

The liquid formulations to be processed by SFD or ASFD in the practice of the invention comprise a non-alum-adsorbed immunogen and may be in any form suitable for atomization, including, for example, a solution, suspension, slurry, or colloid. The liquid formulations may further comprise one or more pharmaceutically acceptable excipients, protectants, solvents, salts, surfactants, and buffering agents. Such excipients are known in the art and may help stabilize the vaccines of the invention. Suitable excipients will be compatible with the immunogen and include, for example, water, saline, carbohydrates, glycerol, ethanol, or the like and combinations thereof. Carbohydrate excipients of particular interest include trehalose, mannitol, dextran, cyclodextrin, inulin USP, and combinations thereof. In certain embodiments, the liquid formulations comprise an immunogen and excipients such as mannitol, trehalose, magnesium chloride, Tween® (e.g., Tween® 80), or any combination thereof. Furthermore, if desired, the liquid formulation may contain auxiliary substances such as wetting or emulsifying agents and pH buffering agents.

Suitable excipients can include free-flowing particulate solids that do not thicken or polymerize upon contact with water, which are innocuous when administered to an individual, and do not significantly interact with the pharmaceutical agent in a manner that alters its pharmaceutical activity. Examples of normally employed excipients include, but are not limited to, proteins such as human and bovine serum albumin, gelatin, or immunoglobulins, monosaccharides such as glucose, xylose, galactose, fructose, D-mannose or sorbose, disaccharides such as lactose, maltose, saccharose, trehalose or sucrose, sugar alcohols such as mannitol, trehalose, sorbitol, xylitol, glycerol, erythritol or arabitol, polymers such as dextran, starch, cellulose or high molecular weight polyethylene glycols (PEG), amino acids or their salts, such as glycine, alanine, glutamine, arginine, lysine or histidine or their salts with alkali or alkaline earth metals such as a sodium, potassium or magnesium salts, or sodium or calcium phosphates, calcium carbonate, calcium sulfite, sodium citrate, citric acid, tartaric acid, pluronics, surfactants, and combinations thereof. Suitable solvents include, but are not limited to, methylene chloride, acetone, methanol, ethanol, isopropanol, and water. Pharmaceutically acceptable salts include, for example, mineral acid salts such as hydrochlorides, hydrobromides, phosphates, sulfates, and the like; and the salts of organic acids such as acetates, propionates, malonates, benzoates, and the like. Chitosan, dermatan sulfate, chondroitin, pectin, and other mucoadhesives may also be used in the practice of the invention, particularly when the vaccine powder formulations are intended for administration by inhalation. Suitable surfactants include but are not limited to Tween® 80, pluronics, and the like. A thorough discussion of pharmaceutically acceptable excipients and auxiliary substances is available in Remington's Pharmaceutical Sciences (18th ed.; Mack Publishing Company, Eaton, Pennsylvania, 1990), which is incorporated herein by reference.

The methods of the invention for preparing a stable vaccine powder formulation comprise the steps of atomizing, freezing, and drying. In accordance with the methods of the invention, the steps of atomizing, freezing, and drying may be performed in a single chamber or apparatus, thereby eliminating the possibility of sample contamination and loss of yield. For example, a liquid formulation may be atomized (i.e., sprayed) into a chamber, wherein the freezing of the atomized formulation and the drying of the frozen particles also occur. Exemplary apparatus for atomizing, freezing, and drying in a single chamber are provided in U.S. Patent Application Publication No. 2003/0180755.

The liquid formulations comprising a non-alum-adsorbed immunogen can be atomized using a variety of methods and devices known in the art. For example, the liquid formulation can be sprayed through a two-fluid nozzle, a pressure nozzle, or a spinning disc nozzle or atomized with an ultrasonic nebulizer, an ink jet printer type nozzle, or a vibrating orifice aerosol generator (VOAG). In some aspects of the invention, the liquid formulation is atomized with a pressure nozzle, such as the BD Accuspray® nozzle. Atomization conditions, including atomization gas flow and gas pressure, liquid flow rate, and nozzle size and type, can be varied, particularly to optimize the size of droplets in the atomized formulation and particle size of the resulting dry powder formulation.

Following atomization of the liquid formulation, the droplets are rapidly frozen to produce solid, frozen particles. In particular embodiments, the droplets are frozen immediately after the atomization step. The droplets may be frozen by introducing the atomized formulation into any cold medium having a temperature below the freezing point of the liquid formulation. As used herein, "introducing the atomized formulation into a cold medium" includes any method for contacting the droplets of the atomized formulation with the cold medium, including but not limited to immersing the droplets in a cold liquid or passing the droplets through a cold gas. The term "cold medium" is broadly defined to include any suitable cold liquid or gas that has a temperature below the freezing point of the liquid formulation. Exemplary cold liquids are known in the art and include liquid nitrogen, argon, and hydrofluoroethers. Compressed liquids, such as compressed fluid carbon dioxide, helium, propane, ethane, or equivalent inert liquids, may also be used in the practice of the present invention. The temperature of the cold liquids used during the freezing step are typically between about -200°C to about -80°C, particularly about -200°C to about -100°C, more particularly about -200°C. Representative gases for use in the freezing step include but are not limited to cold air, nitrogen, helium, and argon and are generally used at a temperature from between about -5°C to about -60°C, more particularly about -20°C to about -40°C. Conventional procedures for obtaining the desired temperature of the cold medium are known in the art. In one embodiment, a liquid formulation comprising the immunogen is atomized through a spray nozzle that is positioned above a vessel (e.g., a metal pan) containing liquid nitrogen. The droplets in the atomized formulation generally freeze immediately upon contact with the cold liquid and are collected and dried.

The solid, frozen particles produced during the freezing step of the claimed methods are dried to produce powder particles of the vaccine. The term "drying" is used herein to refer to the removal of liquid from the frozen particles to produce powder particles having a moisture content of generally less than 20%, 15%, 10%, 5%, or 1% by weight water.

In some embodiments, such as those involving the spray-freeze-drying (SFD) techniques described above and known in the art, the frozen particles are dried by lyophilization (under vacuum) in accordance with methods and devices known in the art. For example, frozen particles may be collected and transferred to a lyophilizer and the excess liquid evaporated off to yield dried powder particles. SFD methods and apparatuses are described in, for example, Jiang et al. (2006) J. Pharm. Sci. 95:80-96; Maa et al. (2003) J Pharm. Sci. 92:319-332; and U.S. Patent Application Publication No. 2003/0180755.

In other aspects of the invention, particularly those involving atmospheric spray-freeze-drying (ASFD) described above, the frozen particles are dried by sublimation in a stream of cold, desiccated gas (e.g., air, nitrogen, or helium) at about atmospheric pressure. As used herein, "about atmospheric pressure" is intended to mean a pressure of approximately 0.5 to five atmospheres, particularly one to three atmospheres, more particularly about one atmosphere of pressure. Methods and apparatus for drying particles at atmospheric pressure are described in U.S. Patent Application Publication No. 2003/0180755 and U.S. Patent No. 4,608,764.

In a particular embodiment, frozen particles are dried in a cold gas at about atmospheric pressure under conditions that promote fluidization of the particles. Particle fluidization during the drying process prevents channeling and agglomeration and permits faster and more complete particle drying. Any method for enhancing the fluidization of the particles during the drying step may be employed in the practice of the invention. For example, the drying step may be performed in the presence of vibration, internals, mechanical stirring, or a combination thereof. The term "internals" is commonly used in the field of industrial process chemistry and is used herein to refer to any physical barrier (e.g., blades, plates, paddles, or other barriers) positioned inside an apparatus or chamber for SFD or ASFD, wherein the physical barrier is used to promote fluidization of particles during the drying process. See U.S. Patent Application No. 2003/0180755.

In certain aspects of the invention, the frozen particles are dried by a combination of processes, such as sublimation in a cold, desiccated gas stream at about atmospheric pressure followed by conventional lyophilization. For example, the frozen particles may be partially dried by contact with the cold gas, collected on a filter or other collection device, and then subjected to lyophilization to further dry the particles. In accordance with the methods of the invention, the drying process may occur, for example, after deposition and collection of the frozen particles or, alternatively, freezing and drying may occur essentially simultaneously. Any method and container or device for collection of frozen, dried, or partially dried powder particles may be used in the invention. In one embodiment, the dried particles may be collected on a filter from which the particles can be removed for further use or in a pan, as in the case of drying by lyophilization. Once collected, the dried powder particles may be transferred to a sterile container suitable for storage of compositions for use in a medical application.

The dried powder particles of the claimed pharmaceutical compositions may be characterized on the basis of a number of parameters, including but not limited to average particle size (also referred to as average geometric particle size or volume mean diameter), range of particle sizes, mean aerodynamic diameter (also referred to as volume mean aerodynamic diameter), particle surface area, and particle morphology (e.g., particle aerodynamic shape and particle surface characteristics). Methods for assessing these parameters are well known in the art. For example, particle size can be assessed by conventional techniques including but not limited to scanning electron microscopy and laser diffraction. The average particle size of the powder can also be measured as a mass mean aerodynamic diameter (MMAD) using conventional techniques such as cascade impaction. Aerodynamic diameter is defined as the product of the actual particle diameter and the square root of the particle's absolute density, as defined herein below and in the art. If desired, automatic particle-size counters can be used (e.g. Aerosizer Counter, Coulter Counter, HIAC Counter, or Gelman Automatic Particle Counter) to ascertain the average particle size. Scanning electron microscopy can be utilized to qualitatively assess particle morphology.

Similarly, the powder particles of the invention may be characterized on the basis of density or a range of particle densities. Actual particle density or "absolute density" can be readily ascertained using known quantification techniques such as helium pycnometry and the like. Alternatively, "tap" density measurements can be used to assess the density of a powder according to the invention. Tap density is defined as the mass of a material that upon packing in a specified manner fills a container to a specific volume, divided by the container volume. Suitable devices are available for determining tap density, for example, the GeoPy^{™} Model 1360, available from the Micromeritics Instrument Corp. The difference between the absolute density and tap density of a powder composition provides information about the composition's percentage total porosity and specific pore volume.

The average particle size of the powder formulations made in accordance with the present methods is generally about 35 µm to about 300 µm, particularly about 80 µm to about 250 µm, more particularly about 80 µm to about 100µm. In some embodiments of the invention, the average particle size is at least 80µm. The average tap density of the powder particles of the invention is typically about 0.01 to about 0.7 g/cm³, particularly about 0.01 to about 0.6 g/cm³, more particularly about 0.02 to about 0.4 g/cm³.

Pharmaceutical compositions comprising a stable vaccine powder formulation, particularly a botulism (e.g., BoNT/A), anthrax (i.e., *B. anthracis),* Staphylococcal enterotoxin (e.g., SEB), and/or plague *(Y. pestis)* vaccine made in accordance with the methods described herein are also encompassed by the present invention. In certain embodiments, the pharmaceutical composition comprises a stable vaccine powder formulation comprising a BoNT immunogen (e.g., BoNT/A, particularly BoNT/A HC), a *B. anthracis* antigen (e.g., *B. anthracis* PA, particularly *B. anthracis* rPA), a Staphylococcal enterotoxin antigen (e.g., SEB, particularly rSEB), and/or a *Y. pestis* antigen (e.g., F1-V). While not intending to limit the invention to specific formulations, in certain embodiments the dried powder comprises (by percent weight) from about 0.0001% to about 10% immunogen and from about 70 to about 99% carbohydrate excipient (e.g., mannitol, trehalose, or dextran).

Pharmaceutical compositions of the invention further include stable vaccine powder formulations that have been reconstituted in a diluent comprising an adjuvant, particularly an aluminum adjuvant or LPS, to form a liquid vaccine for administration to a subject. Methods for producing a reconstituted liquid vaccine are further encompassed by the present invention. In certain embodiments, the methods comprise reconstituting a dried vaccine powder formulation of the invention in a pharmaceutically acceptable carrier comprising an aluminum adjuvant. Aluminum adjuvants are well known in the art and include, for example, aluminum salts such as aluminum hydroxide, aluminum phosphate, and aluminum sulfate. The term "alum" encompasses any aluminum adjuvant. In particular embodiments, the aluminum adjuvant is aluminum hydroxide (e.g., Alhydrogel®). An aluminum adjuvant in a suitable liquid carrier is typically added at the time of reconstitution to a vaccine powder formulation. In other embodiments, the dried vaccine powders, particularly those intended for intranasal administration, are reconstituted in a liquid carrier comprising the adjuvant LPS.

As described above, a "stable" vaccine powder formulation is one in which the dried powder particles can be reconstituted in a diluent, particularly a diluent comprising an aluminum adjuvant or LPS, to produce a reconstituted liquid vaccine that exhibits little or no particle agglomeration, shows no significant decrease in immunogen concentration, retains immunogenicity, maintains antigenicity, and/or exhibits protective efficacy, particularly relative to the corresponding liquid formulation of the vaccine that has not been SFD or ASFD and subsequently reconstituted prior to administration to a subject. These parameters can be assessed using a variety of techniques known in the art (e.g., sedimentation assays, AUSYME assays, analysis of serum antibody concentration, percent survival rates following lethal challenge with a disease-causing pathogen or toxin such as BoNT/A, SEB, or *Y. pestis,* and an anthrax lethal toxin neutralization assay). Results obtained with the reconstituted liquid vaccine may be compared with those obtained using the original liquid formulation comprising the non-alum-adsorbed immunogen adsorbed (i.e., the original liquid formulation prior to atomizing).

In some embodiments, the reconstituted liquid vaccine exhibits little or no particle agglomeration, particularly relative to that of the original liquid formulation. Particle agglomeration may be assessed using such methods as microscopy or sedimentation rate analysis. "Little or no particle agglomeration relative to that of the liquid formulation" indicates, for example, that no significant increase in sedimentation rate is observed with the reconstituted liquid vaccine compared to the sedimentation rate of the original liquid formulation. Furthermore, in certain aspects of the invention, the reconstituted liquid vaccine shows no significant decrease in immunogen concentration relative to the immunogen concentration of the liquid formulation prior to atomization. "No significant decrease in immunogen concentration" is intended to mean that the reconstituted liquid vaccine retains at least about 50, 60, or 70% of the original immunogen concentration, more preferably at least about 80, 85, or 90% of the original immunogen concentration, most preferably at least about 91, 92, 93, 94, 95, 96, 97, 98, 99% or more of the immunogen concentration present in the original liquid formulation. Immunogen concentration may be measured, for example, by an ELISA-based method (e.g., AUSZYME).

As used herein and defined in the art, "antigenicity" is the ability of an antibody to recognize and bind to a protein (e.g., an immunogen). "Immunogenicity" refers to the ability of the protein (i.e., immunogen) to raise an immune response *in vivo* (e.g., in a human or non-human subject). The dried powder vaccine formulations of the invention are generally reconstituted in a diluent comprising an aluminum adjuvant (e.g., aluminum hydroxide) or LPS and retain a substantial level of antigenicity and immunogenicity as compared with that of the corresponding unprocessed liquid vaccine formulation (i.e., not processed by SFD or ASFD). A "substantial level of antigenicity" is intended to mean that the immunogen present in the liquid reconstituted vaccine retains at least about 50, 60, 70, 80, 85, 90, 95, 99% or more antigenicity when compared with that of the corresponding unprocessed liquid vaccine formulation. Antigenicity can be measured by, for example, an ELISA-based assay such as AUSZYME. In certain aspects of the invention, the reconstituted liquid vaccine retains a substantial level of immunogenicity and is therefore able to stimulate an immune response in a subject, particularly an immune response that is substantially the same as that obtained with the corresponding unprocessed liquid vaccine formulation. That is, the immune response achieved by immunization of a subject with the reconstituted liquid vaccine may be greater than, equal to, or at least about 50, 60, 70, 80, 85, 90, 95, 99% or more of the level of immune response obtained with the corresponding unprocessed liquid formulation. Immune response in a subject may be determined by a variety of methods known in the art, including but not limited to measuring serum antibody levels following immunization. Moreover, the "level of protection" or "protective efficacy" obtained with a vaccine of the invention, particularly a vaccine comprising BoNT/A, rPA, rSEB, F1-V, or any combination thereof, may be assessed by the percentage of immunized subjects surviving following exposure to a lethal dose of an immunogen of interest (e.g., a BoNT, such as BoNT/A, *B. anthracis,* or *Y. pestis).* The level of protection obtained with an anthrax vaccine of the invention, particularly a *B*. *anthracis* rPA vaccine, may be determined by, for example, quantifying the neutralizing antibody titer in serum sample, in accordance with methods known in the art.

The term "stable" as applied to the powder compositions herein further indicates that the powders may be subjected to high temperatures, long-term storage, or freeze-thaw cycles and still retain the desired properties with respect to agglomeration, immunogen concentration, immunogenicity, antigenicity, and/or protective efficacy described above.

As discussed above, a stable powder formulation of a vaccine of the invention may be reconstituted in a pharmaceutically acceptable carrier, particularly a pharmaceutically acceptable carrier comprising an adjuvant (e.g., an aluminum adjuvant or LPS), to produce a liquid vaccine formulation suitable for administration to a subject. A "pharmaceutically acceptable carrier" refers to a carrier that is conventionally used in the art to facilitate the storage, administration, or the therapeutic effect of the active ingredient. Pharmaceutically acceptable carriers and methods for formulating pharmaceutical compositions and vaccines are generally known in the art. A thorough discussion of formulation and selection of pharmaceutically acceptable carriers, stabilizers, and isomolytes can be found in Remington's Pharmaceutical Sciences (18th ed.; Mack Publishing Company, Eaton, Pennsylvania, 1990), herein incorporated by reference. Exemplary pharmaceutically acceptable carriers for reconstitution of vaccine powder formulations include a variety of diluents such as physiological saline, buffers, and salts. The term "reconstituted liquid vaccine" is used herein to refer to pharmaceutical compositions comprising a stable vaccine powder formulation that has been reconstituted in a liquid carrier, generally a pharmaceutically acceptable liquid carrier comprising an aluminum adjuvant, to produce a reconstituted liquid vaccine. The methods of the present invention enable the preparation of a dry powder vaccine formulation that is stable and can be readily reconstituted. In particular embodiments, the reconstituted liquid vaccines of the invention exhibit little or no particle agglomeration, display no significant decrease in immunogen concentration, and retain a substantial level of immunogenicity, antigenicity, and/or protective efficacy.

The pharmaceutical compositions of the invention find use in methods of preventing or treating a disease, disorder, condition, or symptoms associated with a particular immunogen. The terms "disease," "disorder," and "condition" will be used interchangeably herein. The polyvalent vaccines of the invention find use in preventing or treating more than one disease. Specifically, the prophylactic and therapeutic methods comprise administration of a therapeutically effective amount of a pharmaceutical composition to a subject. As used herein, "preventing" a disease or disorder is intended administration of a therapeutically effective amount of a pharmaceutical composition of the invention, such as a reconstituted liquid vaccine, to a subject in order to protect the subject from the development of the particular disease or disorder associated with the immunogen, or the symptoms thereof. In some embodiments, a vaccine composition of the invention is administered to a subject such as a human that is at risk for developing the disease or symptoms thereof, including botulism, anthrax, or the plague. Methods of preventing the development of symptoms associated with exposure to a BoNT (e.g., blurred vision, dysphagia, respiratory paralysis, musculoskeletal paralysis, cardiac or respiratory arrest, etc.) are also disclosed. Methods of preventing anthrax or the development of symptoms associated with exposure to *B. anthracis* (e.g., high fever, chest pain, oxygen depletion, secondary shock, increased vascular permeability, systemic hemorrhagic pathology, cardiac or respiratory arrest, etc.) are further encompassed by the present invention. Methods of preventing the development of symptoms associated with exposure, particularly inhalational exposure, to a Staphylococcal enterotoxin (e.g., rSEB) and methods for treating a subject exposed to this agent are also disclosed. Methods of preventing the plague or the development of symptoms associated with exposure to *Y. pestis* in a subject, as well as methods of treating a subject that has the plague or that has been exposed to *Y. pestis* are further envisioned by the present invention. Vaccines directed to potential bioterrorist agents, including but not limited to a BoNT, *B. anthracis, Y. pestis,* and a Staphylococcal enterotoxin, may be prepared, for example, as polyvalent vaccines. Vaccines that provide protective immunity against multiple potential biological weapons may be administered prophylactically to first responders and military personnel or even to the general population in response to a bioterrorist event or threatened bioterrorist event.

By "treating a disease or disorder" is intended administration of a therapeutically effective amount of a pharmaceutical composition of the invention to a subject that is afflicted with the disease or that has been exposed to a pathogen that causes the disease, where the purpose is to cure, heal, alleviate, relieve, alter, remedy, ameliorate, improve, or affect the condition or the symptoms of the disease.

A "therapeutically effective amount" refers to an amount that provides a therapeutic effect for a given condition and administration regimen. In particular aspects of the invention, a "therapeutically effective amount" refers to an amount of a pharmaceutical composition of the invention that when administered to a subject brings about a positive therapeutic response with respect to the prevention or treatment of a subject for a disease. A positive therapeutic response with respect to preventing a disease includes, for example, eliciting an immune response (e.g., the production of antibodies by the subject in a quantity sufficient to protect against development or progression of the disease). Similarly, a positive therapeutic response in regard to treating a disease includes curing or ameliorating the symptoms of the disease.

A therapeutically effective amount can be determined by the ordinary skilled medical worker based on patient characteristics (age, weight, sex, condition, complications, other diseases, etc.). Moreover, as further routine studies are conducted, more specific information will emerge regarding appropriate dosage levels for treatment of various conditions in various patients, and the ordinary skilled worker, considering the therapeutic context, age and general health of the recipient, will be able to ascertain proper dosing. The therapeutically effective amount will be further influenced by the route of administration of the pharmaceutical composition. Generally, for intravenous injection or infusion, and particularly for intradermal administration, the therapeutically effective amount may be lower than that required for intraperitoneal, intramuscular, intranasal, or other route of administration. The dosing schedule may vary, depending on the circulation half-life, and the formulation used. Precise amounts of the pharmaceutical composition required to be administered will depend on the judgment of the practitioner and are peculiar to each individual.

The vaccines of the invention are administered in a manner compatible with the dosage formulation and in such amount as are therapeutically effective and immunogenic (i.e., an antibody-inducing or protective amount, as is desired). The quantity to be administered depends on the subject to be treated, capacity of the subject's immune system to synthesize antibodies, and degree of protection desired. Precise amounts of immunogen required to be administered depend on the judgment of the practitioner and are peculiar to each individual. In a protein vaccine, the amount of protein in each vaccine dose is selected as an amount which induces an immunoprotective response without significant, adverse side effects in typical vaccines. Such amount will vary depending upon which specific immunogen is employed and how it is presented. Optimal amounts of components for a particular vaccine can be ascertained by standard studies involving observation of appropriate immune responses in subjects. Following an initial vaccination, subjects may receive one or several booster immunizations adequately spaced.

The pharmaceutical compositions of the invention can be administered to a subject by a variety of methods known in the art. Any method for administering a composition to a subject may be used in the practice of the invention. Examples of possible routes of administration include pulmonary inhalation, parenteral administration (e.g., intravenous (IV), intramuscular (IM), intradermal (ID), intraperitoneal (IP), subcutaneous (SC) injection or infusion), oral, intranasal (IN), transdermal (topical), transmucosal, and rectal administration. As described above, a pharmaceutical composition comprising a vaccine of interest may be administered as a stable powder (e.g., by pulmonary inhalation, intranasal delivery, or transdermal injection) or as a reconstituted liquid vaccine formulation (e.g. by intranasal delivery or by intradermal, intramuscular, or intravenous injection). In particular, the pharmaceutical compositions comprising a stable powder formulation of a vaccine may be suitable for administration to a subject in powder form by, for example, intranasal delivery, pulmonary inhalation, or transdermal injection. Alternatively, reconstituted liquid vaccines may be administered, for example, intradermally, intravenously, intramuscularly, subcutaneously, intraperitoneally, or intranasally. In certain embodiments of the invention, the vaccines are administered via a minimally invasive method, such as, for example, by intradermal injection through a microneedle or by intranasal inhalation. As used herein, "microneedle" typically includes needles that are 30-gauge or smaller, particularly a 34-gauge needle. Such minimally invasive methods of vaccine administration may permit widespread vaccine administration to the general population by non-medical personnel, which would be particularly advantageous in the event or threat of a bioterrorist attack with a biological weapon such as a BoNT, *B. anthracis, Y. pestis,* or a Staphylococcal enterotoxin (e.g., rSEB).

The prophylactic and therapeutic methods of the present invention are not intended to be limited to particular subjects. A variety of subjects, particularly mammals, are contemplated. Subjects of interest include but are not limited to humans, dogs, cats, horses, pigs, cows, and rodents. In particular embodiments, the subject is a human, more particularly a human patient at risk for developing the disease associated with the specific antigen.

In accordance with the present invention there may be employed conventional molecular biology, microbiology, and recombinant DNA techniques within the skill of the art. Such techniques are explained fully in the literature. See, e.g., Sambrook et al., Molecular Cloning: A Laboratory Manual (1989); Current Protocols in Molecular Biology, Volumes I-III (Ausubel, R. M., ed. (1994)); Cell Biology: A Laboratory Handbook, Volumes I-III (J. E. Celis, ed. (1994)); Current Protocols in Immunology, Volumes I-III (Coligan, J. E., ed. (1994)); Oligonucleotide Synthesis (M.J. Gait ed. 1984); Nucleic Acid Hybridization (B.D. Hames & S.J. Higgins eds. (1985)); Transcription And Translation (B.D. Hames & S.J. Higgins, eds. (1984)); Animal Cell Culture (R.I. Freshney, ed. (1986)); Immobilized Cells And Enzymes (IRL Press, (1986)); B. Perbal, A Practical Guide To Molecular Cloning (1984).

The article "a" and "an" are used herein to refer to one or more than one (i.e., to at least one) of the grammatical object of the article. By way of example, "an element" means one or more element.

Throughout the specification the word "comprising," or variations such as "comprises" or "comprising," will be understood to imply the inclusion of a stated element, integer or step, or group of elements, integers or steps, but not the exclusion of any other element, integer or step, or group of elements, integers or steps.

The following examples are offered by way of illustration and not by way of limitation.

### EXPERIMENTAL

### Example 1: Analysis of SFD Y. pestis Vaccine Formulations (Study #1) Preparation of SFD Y. pestis F1-V Vaccine Formulations

F1-V protein was provided by the National Institutes of Allergy and Infectious Diseases and formulated with various excipients and adjuvants as a liquid suspension (i.e., without SFD processing) or as a dried powder processed by SFD. The SFD F1-V vaccine powder formulations were reconstituted in a liquid suspension of Alhydrogel® (i.e., aluminum hydroxide) prior to immunization of the mice, as described below for Groups 3 and 6.
**Group 1**: In a 2-ml vial 0.133 ml of F1-V protein (at a concentration of 1.5 mg/ml), 0.1 ml of Alhydrogel®, and 0.767 ml of buffer (i.e., 10 mM NaCl, 20 mM arginine, and 1 mM cystine, pH 9.9) were mixed until a uniform suspension was formed and used for intramuscular (IM) immunization of test mice.
**Group 2**: In a 10-ml vial 114.2 mg of mannitol, 12.6 mg of trehalose, 0.466 ml of the F1-V solution, 0.35 ml of Alhydrogel®, and 2.685 ml of buffer (i.e., 10 mM NaCl, 20 mM arginine, and 1 mM cystine, pH 9.9) were mixed until a uniform suspension was formed. A 1.0 ml aliquot was removed and used as-is in liquid form.
**Group 3**: In a 10-ml vial 195.6 mg of mannitol, 21.7 mg of trehalose, 0.8 ml of the F1-V solution, 0.602 ml of water suitable for injection, and 4.614 ml of buffer (i.e., 10 mM NaCl, 20 mM arginine, 1 mM cystine, pH 9.9) were mixed until a uniform solution was formed. This solution was sprayed into liquid nitrogen using an Accuspray® nozzle attached to a 1-ml syringe. This frozen sample was placed in a shelf lyophilizer pre-cooled to -45°C and dried under vacuum. As noted above, the resulting SFD F1-V powder vaccine was reconstituted in a suspension of Alhydrogel® prior to use in IM injections.
**Group 4**: In a 2-ml vial 0.284 ml of buffer exchanged F1-V (at a concentration of 0.703 mg/ml), 0.1 ml Alhydroge145, and 0.616 ml of buffer (i.e., 10 mM Nad, 20 mM arginine, 1 mM cystine, pH 9.9) were mixed until a uniform suspension was formed and used for IM immunization of test mice.
**Group 5**: In a 10-ml vial 125.8 mg of mannitol, 13.9 mg of trehalose, 0.994 ml of the F1-V solution, 0.35 ml of Alhydrogel45, and 2.156 ml of buffer (10 mM Nad, 20 mM arginine, 1 mM cystine, pH 9.9) were mixed until a uniform suspension was formed. A 1.0 ml aliquot was removed and used as-is in liquid form.
**Group 6**: In a 10-ml vial 195.6 mg of mannitol, 21.7 mg of trehalose, 0.8 ml of the F1-V solution, 0.602 ml of water suitable for infection, and 4.614 ml of buffer (10 mM NaCl, 20 mM arginine, 1 mM cystine, pH 9.9) were mixed until a uniform solution was formed. This solution was sprayed into liquid nitrogen using an Accuspray® nozzle attached to a 1-ml syringe. This frozen sample was placed in a shelf lyophilizer pre-cooled to -45°C and dried under vacuum. Again, the resulting SFD F1-V powder vaccine was reconstituted in a suspension of Alhydrogel® prior to use in IM injections.

A summary of the *Y. pestis* F1-V vaccines test groups of Study #1 is provided in Table 1:

**Table 1: Summary of Y. pestis F1-V Vaccine Test Groups in Study #1**

| **Group** | **Dose** (µg) | **Formulation** | **Processing** |
|---|---|---|---|
| 1 | 10 | AI(OH)₃ | Liquid Suspension |
| 2 | 10 | Al(OH)₃†-/Mannitol/Trehalose | Liquid Suspension |
| 3 | 10 | Al(OH)₃/Mannitol/Trehalose | SFD |
| 4 | 3.3 | Al(OH)₃/Tween® 80/MgCl₂ | Liquid Suspension |
| 5 | 3.3 | Al(OH)₃/Mannitol/ Trehalose/Tween® 80/MgCl₂ | Liquid Suspension |
| 6 | 3.3 | Al(OH)₃†/Mannitol/ Trehalose/Tween® 80/MgCl₂ | SFD |

| | | | |
|---|---|---|---|
| †**Dried vaccine powder reconstituted in suspension of Alhydrogel® prior to IM injection.** | | | |

### Immunizations

6-8 week old female Swiss Webster mice were housed and immunized with the above vaccine formulations. Ten mice were used per test group. Each mouse was immunized intramuscularly with 50 µl (25 µl per site) of the specified vaccine formulation at days 0 and 28 and at a dose of 3.3 µg or 10 µg of F1-V. Blood was collected on days 0, 14, 28 and 42 to assess serum antibody titers in accordance with standard ELISA methods.

### Results

The results obtained with the *Y. pestis* F1-V vaccine formulations of Groups 1-6 are presented in Figure 1. The data shown in this figure demonstrate that the SFD F1-V vaccine powder reconstituted in Alhydrogel® resulted in antibody production at levels similar to those obtained following immunization with the corresponding unprocessed liquid vaccine suspensions.

### Example 2: Analysis of SFD Y pestis Vaccine Formulations (Study #2) Liquid F1-V Vaccine Formulations

F1-V protein was provided by the National Institutes of Allergy and Infectious Diseases and formulated with various excipients and adjuvants as a liquid suspension (i.e., without SFD/ASFD processing) or processed by SFD or ASFD to produce a dried vaccine powder. Pressure diafiltration was used to prepare 4.5 ml of an F1-V solution in a buffer containing 20 mM Tris, 50 mM MgCl₂, and 2% Tween® 80 (pH 7.4 and a F1-V final concentration of 0.609 mg/ml). Mannitol, trehalose, and Alhydrogel® were added to this solution to produce the liquid vaccine suspensions for intramuscular (IM) and intradermal (ID) injection. Mannitol, trehalose, and lipopolysaccharide (LPS) were added to produce the liquid vaccine suspensions for intranasal (IN) delivery.

### Preparation of SFD Y. pestis F1-V Vaccine Formulations

In a 10-ml vial 381.5 mg of mannitol, 42.4 mg of trehalose, 4.5 ml of the F1-V solution, and 0.99 ml water suitable for injection were mixed until a uniform solution was formed. The solution was sprayed into liquid nitrogen using an Accuspray® nozzle attached to a 1-ml syringe. The resulting frozen sample was placed in a shelf lyophilizer pre-cooled to -45°C and dried under vacuum. The SFD vaccine powder formulations were reconstituted in an LPS solution prior to IN delivery or in an Alhydrogel® suspension prior to IM/ID injection of test mice.

### Preparation of ASFD Y. pestis F1-V Vaccine Formulations

In a 20-ml vial 1.827 g of mannitol, 203 mg trehalose, and 15 ml of F1-V solution (at 0.609 mg/ml) was mixed until a uniform solution was formed. The solution was sprayed into liquid nitrogen using an Accuspray® nozzle attached to a 1-ml syringe, and the frozen slurry was transferred into the ASFD drying chamber that had been pre-cooled with liquid nitrogen. Drying progressed under control of a LabVIEW (LabVIEW 7.1, National Instruments Corporation, Austin, TX) program. This LabVIEW program was used to monitor system parameters and control variables such as temperature and gas flow rate. Recovered vaccine powder was stored at 4°C until use and then reconstituted in an LPS solution prior to IN delivery or in an Alhydrogel® suspension prior to IM/ID administration of test mice.

A summary of the *Y. pestis* F1-V vaccines test groups of Study #2 is provided in Table 2.

**Table 2: Summary of Y. pestis F1-V Vaccine Test Groups in Study #2**

| **Group** | **Method of** | **Formulation** | **Adjuvant** | **Excipient(s)** | **F1-V Dose** | **Immunization** |
|---|---|---|---|---|---|---|
| 1 | IM | Liquid | 0.3% Al(OH)₃ | Mannitol/Trehalose/Tween® 80/MgCl₂ | 10 | d0, d28 |
| 2 | IM | SFD | †0.3% Al(OH)₃ | Mannitol/Trehalose/Trween® 80/MgCl₂ | 10 | d0, d28 |
| 3 | IM | ASFD | †0.3% Al(OH)₃ | Mannitol/Trehalose/Tween® 80/MgCl₂ | 10 | d0, d28 |
| 4 | ID | Liquid | 0.3% Al(OH)₃ | Mannitol/Trehalose/Tween® 80/MgCl₂ | 10 | d0, d28 |
| 5 | ID | SFD | †0.3% Al(OH)₃ | Mannitol/TrehaloselTween® 80/MgCl₂ | 10 | d0, d28 |
| 6 | ID | ASFD | †0,3% Al(OH)₃ | Mannitol/Trehalose/Tween® 80/MgCl₂ | 10 | d0, d28 |
| 7 | IN | Liquid | LPS | Mannitol/Trehalose/Tween® 80/MgCl₂ | 10 | d0, d28 |
| 8 | IN | SFD | †LPS | Mannitol/Trehalose/Tween®80/MgCl₂ | 10 | d0 d28 |
| 9 | IN | ASFD | †LPS | Mannitol/Trehalose/Tween® 80/MgCl₂ | 10 | d0, d28 |
| 10 | IN | Liquid | LPS | Mannitol/Trehalose/Tween® 80/MgCl₂ | 10 | d0, d14, d28 |
| 11 | IN | SFD | †LPS | Mannitol/Trehatose/Tween® 80/MgCl₂ | 10 | d0, d14, d28 |
| 12 | IN | ASFD | †LPS | Mannitol/Trehalose/Tween® 80/MgCl₂ | 10 | d0, d14, d28 |
| 13 | IN | Liquid | LPS | Mannitol/Trehalose/MgCl₂ | 10 | d0, d28 |
| 14 | IM | Liquid (antigen-only control) | None | Mannitol/Trehalose/Tween® 80/MgCl₂ | 10 | d0, d28 |
| 15 | ID | (an Liquid control) (antigen-only control) | None | Mannitol/TrehaloselTween® 80/MgCl₂ | 10 | d0,d28 d0, d28 |
| 16 | IN | Liquid (antigen-only control) | None | Mannitol/Trehalose/Tween® 80/MgCl₂ | 10 | d0, d28 |
| 17 | IN | Liquid (antigen-only control) | None | Mannitol/Trehalose/Tween® 80/MgCl₂ Mannitol/Trehalose/Tween® 80/MgCl₂ | 10 | d0, d14, d28 |
| 18 | None | None (naive ctrl) | None | None | 0 | NA |

| | | | | | | |
|---|---|---|---|---|---|---|
| **†Al(OH)₃ (i.e., Alhydrogel®) or LPS added at time of reconstitution of dried vaccine powder.** | | | | | | |

### Immunizations, Analysis of Antibody Titers, and Lethal Challenge with Y. pestis

6-8 week old female Swiss Webster mice were housed and immunized with the above vaccine formulations prior to *Y. pestis* lethal challenge. Ten mice were used per test group. Each mouse was immunized intramuscularly or intradermally (via a microneedle) with 50 µl (25 µl per site) or immunized intranasally with 30 µl (25 µl per nostril) of the specified vaccine formulation at days 0 and 28. A subset of the mice immunized intranasally received a third dose of the vaccine on day 14, as indicated in Table 2 above. Mice immunized IM, ID, or IN with a liquid formulation of F1-V without any adjuvant (e.g., LPS or Alhydrogel®) and un-immunized mice were used as controls.

Blood was collected on days 0, 28 and 42 to assess antibody titers. Specifically, serum samples were analyzed for F1-V, F1, or V antigen-specific IgG titers in accordance with standard ELISA methods known in the art. Mice were then challenged subcutaneously (SC) with a lethal dose of *Y. pestis* (i.e., 1000 x LD₅₀) on day 56, and the survival rates for each vaccine test group were determined for twenty-five days post-challenge.

### Results

The results obtained with the *Y. pestis* F I -V vaccine formulations of Groups 1-18 with respect to antibody titers and survival rates are presented in Figures 2-4. In summary, the data demonstrate that the reconstituted SFD and ASFD F1-V vaccine formulations elicited similar serum antibody responses and survival rates following lethal challenge with *Y. pestis* in mice immunized intramuscularly relative to those obtained with the corresponding unprocessed liquid F1-V vaccine formulation. The serum IgG titers and percent survival rates were slightly lower in mice immunized intradermally or intranasally with ASFD formulations of the F1-V vaccine when compared to results obtained with either the corresponding unprocessed liquid or SFD F1-V vaccine. In general, however, ID delivery of the reconstituted SFD or ASFD FI-V vaccine produced serum antibody titers and survival rates comparable to those observed with IM injection. IN delivery of a F1-V vaccine typically required administration of at least one additional dose in order to achieve the level of protection against *Y. pestis* observed with either IM or ID injection.

Serum antibody titers for the F1, V, and F1-V antigens were measured and correlated with survival rates following lethal challenge with *Y. pestis.* Specifically, antibody titers for each antigen for the test group exhibiting the highest survival rate (i.e., Group 4), an intermediate survival rate (i.e., Group 7), and the lowest survival rate (i.e., Groups 9 and 18) were compared, as shown in Table 3. The results indicate that the anti-F1 antigen antibody titer is a better predictor of protection from *Y. pestis* challenge than antibody titers for either the V or F1-V antigen.

**Table 3: F1, V, and F1-V Antibody Titers from Test Groups with the Highest, Intermediate, and Lowest Percent Survival Rates**

| Group | 4 | | | 7 | | | 9 | | | 18 (Naïve) | | |
|---|---|---|---|---|---|---|---|---|---|---|---|---|
| Method of Delivery | ID | | | IN | | | IN | | | NA | | |
| Formulation | Liquid | | | Liquid | | | ASFD | | | NA | | |
| Adjuvant | Al(OH)₃ | | | LPS | | | LPS† | | | NA | | |
| Excipients | Mannitol/Trehalose/Tween® 80/MgCl₂ | | | Mannitol/Trehalose/Tween® 80/MgCl₂ | | | Mannitol/Trehalose/Tween® 80/MgCl₂ | | | NA | | |
| Immunization Days | Days 0 and 28 | | | Days 0 and 28 | | | Days 0 and 28 | | | NA | | |
| % **Survival** | 100% | | | 50% | | | 11% | | | 10% | | |
| Antibody Type | F1 | V | F1-V | F1 | V | F1-V | F1 | V | F1-V | F1 | V | F1-V |
| 1 | 102400 | 409600 | 204800 | 1600 | 102400 | 102400 | <50 | 204800 | 102400 | | | <50 |
| 2 | 3200 | 409600 | 204800 | <50 | >819200 | 51200 | 100 | 25600 | 12800 | | | <50 |
| 3 | 12800 | 409600 | 204800 | <50 | 102400 | 102400 | <50 | <50 | <50 | | | <50 |
| 4 | 102400 | 1638400 | 819200 | 1600 | 204800 | 204800 | 100 | 3200 | 800 | | | <50 |
| 5 | 12800 | 819200 | 409600 | <50 | >819200 | 51200 | 1600 | 409600 | 204800 | | | <50 |
| 6 | 6400 | 819200 | 409600 | <50 | 409600 | 409600 | <50 | >6400 | 6400 | | | <50 |
| 7 | 204800 | 819200 | 409600 | 100 | 819200 | 819200 | 400 | 819200 | 409600 | | | <50 |
| 8 | 51200 | 819200 | 819200 | 1600 | >204800 | 6400 | <50 | 400 | 100 | | | <50 |
| 9 | 25600 | 409600 | 204800 | 6400 | 409600 | 204800 | <50 | 200 | 50 | | | <50 |
| 10 | 204800 | 3276800 | 3276800 | <50 | 409600 | 204800 | Dead | Dead | Dead | | | <50 |
| Pooled sample | | | | | | | | | | <50 | <50 | |

| | | | | | | | | | | | | |
|---|---|---|---|---|---|---|---|---|---|---|---|---|
| **†LPS added at time of reconstitution of dried vaccine powder.** | | | | | | | | | | | | |

### Example 3: Analysis of SFD Polyvalent Vaccine Formulations

### Preparation of SFD Polyvalent Vaccine Formulations

A polyvalent vaccine comprising four antigens for potential bioterror agents (i.e., rPA, rSEB, BoNT/A, and F1-V) was prepared as a single formulation. In particular, solutions of F1-V (pH 9.0) were first processed by pressure diafiltration to exchange the buffer/excipients to 20 mM Tris, 50 mM MgCl₂, and 2% Tween® 80 (pH 7.4). Solutions of the other three antigens of interest (i.e., rPA, BoNT/A, and rSEB), were added to this F1-V solution to create the polyvalent solution. For the corresponding unprocessed liquid vaccines used in this experiment, mannitol, trehalose, and Alhydrogel® were added to this polyvalent solution to produce liquid vaccine suspensions. Mannitol and trehalose were also added to the polyvalent solutions that were to be processed by SFD. The suspensions were spray-freeze dried to produce a dried powder, essentially as described above in Examples 1 and 2. The resulting SFD powder was reconstituted in a suspension of Alhydrogel® for injection at the time of immunization. Evaluation of the reconstituted vaccine powder by reverse phase HPLC (RP-HPLC) indicated that all four antigens were soluble upon reconstitution and could be completely recovered following SFD. See Figure 5.

The initial and final concentrations of each antigen are listed below:
Initial antigen concentration (as received from vendor):
   rPA: 2.5 mg/mL rPA
   rSEB: 3.4 mg/mL
   BoNT/A: 0.18 mg/mL
   F1-V: 600 µg/mL
Final antigen concentration (in dosed liquid and reconstituted SFD vaccine formulations):
   rPA: 200 µg/mL
   rSEB: 400 µg/mL
   BoNT/A: 20 µg/mL
   F1-V: 200 µg/mL

### Immunizations

Female BALB/c mice were immunized with a liquid or reconstituted SFD polyvalent vaccine formulation. Liquid monovalent vaccine formulations (i.e., comprising only one of the rPA, rSEB, BoNT/A, or F1-V antigens) were used as controls. Ten mice were used for each test group, and each mouse was immunized intramuscularly (IM) with 50 µl (25 µl per site) of the specified vaccine formulation at days 0 and 28. The details of the test groups are summarized below in Table 4. Pre-bleed samples were collected from naive, un-immunized mice. All test groups were bled on days 14, 28, and 42, and serum samples were analyzed by standard ELISA methods to determine antibody titers for each antigen (i.e., rPA, rSEB, BoNTA and F1-V.)

**Table 4: Summary of Polyvalent Vaccine Test Groups**

| **Group** | **Method of Delivery** | **rPA Dose (µg)** | **rSEB Dose (µg)** | **BoNT Dose (µg)** | **F1-V Dose (µg)** | **Alhydrogel Dose** % **Al(OH)₃*** | **Formulation** |
|---|---|---|---|---|---|---|---|
| 1 | **IM** | **10** | **20** | **1** | **10** | **0.5** | **Liquid** |
| 2 | IM | 10 | 20 | 1 | 10 | 0.5 | Reconstituted SFD† |
| 3 | IM | 10 | 0 | 0 | 0 | 0.5 | Liquid |
| 4 | IM | 0 | 20 | 0 | 0 | 0.5 | Liquid |
| 5 | IM | 0 | 0 | 1 | 0 | 0.5 | Liquid |
| 6 | IM | 0 | 0 | 0 | 10 | 0.5 | Liquid |
| 7 | IM | 0 | 0 | 0 | 0 | 0 | Liquid (naïve control) |

| | | | | | | | |
|---|---|---|---|---|---|---|---|
| ***Calculation based on amount in the final suspension for injection.** **† Alhydrogel® added at time of reconstitution.** | | | | | | | |

### Results

The antibody titer results obtained with pooled serum samples from mice immunized with the various vaccine formulations of Groups 1-7 on days 14, 28, and 42 are presented below in Tables 5-7, respectively. Mice immunized with liquid or reconstituted SFD polyvalent vaccine formulations containing the rPA, BoNT/A, rSEB, and F1-V antigens (i.e., test groups 1 and 2) generated an immune response to all four of the antigens. The antibody titers measured for each antigen in mice immunized with the polyvalent vaccine formulations were similar to those determined for each antigen in mice immunized with the liquid monovalent control vaccine formulations (i.e., test groups 3-6), indicating that the combination of the four antigens in the polyvalent vaccine did not adversely affect the immunogenicity of each antigen. Furthermore, mice immunized with the SFD polyvalent vaccine reconstituted in a suspension of Alhydrogel® (i.e., test group 2) exhibited a similar elevated pooled serum titer as that observed with mice immunized with the corresponding liquid polyvalent vaccine (i.e., test group 1), demonstrating that the SFD process did not decrease the immunogenicity of the various antigens. Similar antibody titer results were obtained when antibody titers for individual animals were analyzed. See Figure 6.

To confirm that the measured antibody titers were specific to the immunizing antigen, mice immunized with the liquid monovalent vaccine controls were also screened for antibodies to antigens that were not present in the monovalent vaccine. For example, antibody titers for rSEB, rPA, and F1-V were analyzed following immunization with the liquid BoNT/A monovalent vaccine. Antibody titers generated by the monovalent vaccines were found to be antigen-specific at each time point, and no significant antibody levels were observed for the other antigens not present in the monovalent vaccine. Moreover, pooled group serum from un-immunized, naive mice (group 7) failed to produce detectable antibody titers when screened against all four antigens, indicating that none of the mice in this study had a pre-existing immunity to any of the immunogens tested and further demonstrating that the titers generated in the immunized groups were specific to the immunizing antigen(s).

**Table 5: Summary of Antibody Titers for Polyvalent Vaccine Test Groups (Day 14)**

| | | | | | Antigen Screened (Antibody Titers) | | | |
|---|---|---|---|---|---|---|---|---|
| **Group** | **Formulation** | **Al(OH)₃ %** | **Method of Delivery** | **rPA/rSEB/BoNT/A/F1-V Dose (µg)** | **F1-V** | **SEB** | **rPA** | **BoNT/A** |
| 1 | Liquid | 0.5 | IM | 10/20/1/10 | 6400 | 200 | 50 | <50 |
| 2 | Reconstituted | 0.5 | IM | 10/20/1/11 | 6400 | <50 | <50 | <50 |
| 3 | Liquid | 0.5 | IM | 10/0/0/0 | <50 | <50 | <50 | <50 |
| 4 | Liquid | 0.5 | IM | 0/20/0/0 | <50 | <50 | 50 | <50 |
| 5 | Liquid | 0.5 | IM | 0/0/1/0 | <50 | <50 | <50 | <50 |
| 6 | Liquid | 0.5 | IM | 0/0/0/10 | 3200 | <50 | <50 | <50 |
| 7 | Liquid (naïve control) | 0.5 | IM | 0/0/0/0 | <50 | <50 | <50 | <50 |
| 8 | Liquid | 0.25 | IM | 10/20/1/10 | 6400 | <50 | <50 | <50 |
| 9 | Liquid | 0.25 | ID | 10/20/1/10 | 6400 | 50 | 50 | <50 |

| | | | | | | | | |
|---|---|---|---|---|---|---|---|---|
| **† Alhydrogel® added at time of reconstitution.** | | | | | | | | |

**Table 6: Summary of Antibody Titers for Polyvalent Vaccine Test Groups (Day 28)**

| | | | | | Antigen Screened (Antibody Titers) | | | |
|---|---|---|---|---|---|---|---|---|
| **Group** | **Formulation** | **AI(OH)₃** % | **Method of** Delivery | **rPA/rSEB/BoNT/A/F1-V** Dose (µg) | **F1-V** | **SEB** | **rPA** | **BoNT/A** |
| 1 | Liquid | 0.5 | IM | 10/20/1/10 | 25600 | 6400 | 1600 | 1600 |
| 2 | Reconstituted SFD† | 0.5 | IM | 10/20/1/11 | 25600 | 6400 | 800 | 800 |
| 3 | Liquid | 0.5 5 | IM | 10/0/0/0 | <100 | <100 | 1600 | <50 |
| 4 | Liquid | 0.5 | IM | 0/20/0/0 | <100 | 1600 | <100 | <50 |
| 5 | Liquid | 0.5 | IM | 0/0/1/0 | <100 | <100 | <100 | 1600 |
| 6 | Liquid | 0.5 | IM | 0/0/0/10 | 12800 | <100 | <100 | <50 |
| 7 | Liquid (naïve control) | 0.5 | IM | 0/0/0/0 | <100 | <50 | <50 | <50 |
| 8 | Liquid | 0.25 | IM | 10/20/1/10 | 12800 | 3200 | 200 | 400 |
| 9 | Liquid | 0.25 | ID | 10/20/1/10 | 12800 | 1600 | 400 | 400 |

| | | | | | | | | |
|---|---|---|---|---|---|---|---|---|
| **†Alhydrogel® added at time of reconstitution.** | | | | | | | | |

**Table 7: Summary of Antibody Titers for Polyvalent Vaccine Test Groups (Day 42)**

| | | | | | Antigen Screened (Antibody Titers) | | | |
|---|---|---|---|---|---|---|---|---|
| **Group** | **Formulation** | **Al(OH)₃ %** | **Method of Delivery** | **rPA/rSEB/BoNT/A/F1-V Dose (µg)** | **F1-V** | **rSEB** | **rPA** | **BoNT/A** |
| 1 | Liquid | 0.5 | IM | 10/20/1/10 | 102400 | 51200 | 51200 | 25600 |
| 2 | Reconstituted SFD† | 0.5 | IM | 10/20/1/11 | 204800 | 51200 | 51200 | 25600 |
| 3 | Liquid | 0.5 | IM | 10/0/0/0 | <200 | <200 | 12800 | <50 |
| 4 | Liquid | 0.5 | IM | 0/20/0/0 | <200 | 6400 | <200 | <50 |
| 5 | Liquid | 0.5 | IM | 0/0/1/0 | <200 | <200 | <200 | 6400 |
| 6 | Liquid | 0.5 | IM | 0/0/0/10 | 51200 | <200 | <200 | <50 |
| 7 | Liquid (naïve control) | 0.5 | IM | 0/0/0/0 | <100 | <100 | <100 | <50 |
| 8 | Liquid | 0.25 | IM | 10/20/1/10 | 102400 | 25600 | 51200 | 25600 |
| 9 | Liquid | 0.25 | ID | 10/20/1/10 | 102400 | 12800 | 25600 | 25600 |

| | | | | | | | | |
|---|---|---|---|---|---|---|---|---|
| **† Alhydrogel® added at time of reconstitution.** | | | | | | | | |

All publications and patent applications mentioned in the specification are indicative of the level of those skilled in the art to which this invention pertains. All publications and patent applications are herein incorporated by reference to the same extent as if each individual publication or patent application was specifically and individually indicated to be incorporated by reference.

Although the foregoing invention has been described in some detail by way of illustration and example for purposes of clarity of understanding, it will be obvious that certain changes and modifications may be practiced within the scope of the appended claims.

## Claims

1. A pharmaceutical composition comprising a stable powder formulation of vaccine particles comprising at least one non-alum adsorbed immunogen selected from a Botulinum neurotoxin (BoNT), a *Yersinia pestis* antigen, and a combination thereof.

2. A pharmaceutical composition according to claim 1 further comprising a *Bacillus anthracis* antigen, wherein the antigen is *B. anthracis* rPA.

3. A pharmaceutical composition according to claim 1 or claim 2 further comprising a Staphylococcal enterotoxin, wherein the enterotoxin is recombinant *Staphylococcus* enterotoxin B (rSEB).

4. A pharmaceutical composition according to any one of claims 1 to 3 comprising a plurality of non-alum-adsorbed immunogens, wherein the plurality of immunogens comprises BoNT/A, F1-V, rPA and rSEB.

5. A pharmaceutical composition according to any one of the preceding claims wherein the dried vaccine powder particles are reconstituted in a pharmaceutically acceptable carrier.

6. A method for preparing a stable powder formulation of a vaccine comprising:
a) providing a liquid formulation comprising at least one immunogen selected from a Botulinum neurotoxin (BoNT) and a *Yersinia pestis* antigen, wherein the liquid formulation does not contain an aluminium adjuvant;
b) atomizing the liquid formulation to produce an atomized formulation;
c) freezing the atomized formulation to produce frozen particles; and
d) drying the frozen particles to produce dried powder particiles.

7. A method according to claim 6, wherein the drying is performed at about atmospheric pressure and in the presence of vibration, internals, mechanical stirring, or a combination thereof.

8. A method according to claim 6 or claim 7 or a pharmaceutical composition according to any one of claims I to 5, wherein the BoNT is BoNT/A.

9. A method according to any one of claims 6 to 8 or a pharmaceutical composition according to any one of claims I to 5, wherein the *Y. pestis* immunogen is an F1-V fusion portein (F1-V).

10. A method according to any one of claims 6 to 9, wherein the liquid formulation further comprises a *Bacillus anthracis* antigen, wherein the antigen is *B. anthracis* rPA.

11. A method according to any one of claims 6 to 10, wherein the liquid formulation further comprises a Staphylococcal enterotoxin, wherein the enterotoxin is recombinant *Staphylococcus* enterotoxin B (rSEB).

12. A method according to any one of claims 6 to 11, wherein the liquid formulation comprises a plurality of immunogens, wherein the plurality of immunogens comprises rPA, BoNT/A, rSEB, and F1-V.

13. A method according to any one of claims 6 to 12, wherein the liquid formulation further comprises at least one excipient selected from mannitol, trehalose, dextran, and a combination thereof.

14. A method according to any one of claims 6 to 13, wherein the liquid formulation further comprises at least one adjuvant.

15. A method according to claim 14, wherein the adjuvant is lipopolysaccharide (LPS).

16. A method according to any one of claims 6 to 15 or a pharmaceutical composition according to any one of claims 1 to 5, wherein the average particle size in the range of from 35 µm to 300 µm.

17. A method according to any one of claims 6 to 16 or a pharmaceutical composition according to any of claims 1 to 5 and 16, wherein the average particle size in the range of from 80 µm to 100 µm.

18. A method according to any one of claims 6 to 17 or a pharmaceutical composition according to any one of claims 1 to 5, 16 and 17, wherein the dried powder particles have an average tap density in the range of from 0.01 g/cm³ to 0.7 g/cm³, from 0.01 g/cm³ to 0.6 g/cm³, or from 0.02 g/cm³ to 0.4 g/cm³.

19. A pharmaceutical composition comprising a stable powder formulation of vaccine particles comprising at least one non-alum adsorbed immunogen selected from a Botulinum neurotoxin (BoNT), a *Yersinia pestis* antigen, and a combination thereof, for use in preventing development of a disease or symptoms associated with exposure to *B*. *anthracis,* a BoNT, *Y. pestis,* a Staphylococcal enterotoxin, or a combination thereof.

20. A pharmaceutical composition comprising a stable powder formulation of vaccine particles comprising at least one non-alum adsorbed immunogen selected from a Botulinum neurotoxin (BoNT), a *Yersinia pestis* antigen, and a combination thereof, for use in treating a subject exposed to *B. anthracis,* a BoNT, *Y*. *pestis,* a Staphylococcal enterotoxin, or a combination thereof.

21. A pharmaceutical composition according to claim 19 or claim 20 wherein the pharmaceutical composition is as defined in any one of claims 2 to 5, 8, 9 and 16 to 18.

22. Use of a pharmaceutical composition comprising a stable powder formulation of vaccine particles comprising at least one non-alum adsorbed immunogen selected from a Botulinum neurotoxin (BoNT), a *Yersinia pestis* antigen and a combination thereof, in the manufacture of a medicament for preventing development of a disease or symptoms associated with exposure to *B. anthracis,* a BoNT, *Y. pestis,* a Staphylococcal enterotoxin or a combination thereof.

23. Use of a pharmaceutical composition comprising a stable powder formulation of vaccine particles comprising at least one non-alum adsorbed immunogen selected from a Botulinum neurotoxin (BoNT), a *Yersinia pestis* antigen and a combination thereof, in the manufacture of a medicament for treating a subject exposed to *B. anthracis,* a BoNT, *Y. pestis,* a Staphylococal enterotoxin or a combination thereof.
